(19) Europäisches Patentamt
European Patent Office
Office européen des brevets

(11) EP 4 554 551 B1

(12) **EUROPEAN PATENT SPECIFICATION**

(45) Date of publication and mention of the grant of the patent:
**11.03.2026 Bulletin 2026/11**

(21) Application number: **24742868.3**

(22) Date of filing: **09.07.2024**

(51) International Patent Classification (IPC):
***A61K 8/37*** *(2006.01)* ***A61Q 17/00*** *(2006.01)*
***A61Q 15/00*** *(2006.01)* ***A01N 37/12*** *(2006.01)*
***A01P 1/00*** *(2006.01)* ***A61P 17/16*** *(2006.01)*
***C11D 3/20*** *(2006.01)* ***C11D 3/48*** *(2006.01)*
***C11D 3/00*** *(2006.01)* ***C11D 7/26*** *(2006.01)*

(52) Cooperative Patent Classification (CPC):
**A61K 8/375; A01N 37/12; A01P 1/00; A61P 17/16; A61Q 15/00; A61Q 17/005; C11D 3/0068; C11D 3/2093; C11D 3/48; C11D 7/266;**
A61K 2800/524

(86) International application number:
**PCT/EP2024/069327**

(87) International publication number:
**WO 2025/012263 (16.01.2025 Gazette 2025/03)**

(54) **3-HYDROXYPROPYLESTERS AS ANTIMICROBIAL AGENTS**
3-HYDROXYPROPYLESTER ALS ANTIMIKROBIELLE WIRKSTOFFE
3-HYDROXYPROPYLESTERS UTILISÉS COMME AGENTS ANTIMICROBIENS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **10.07.2023 PCT/EP2023/068983**

(43) Date of publication of application:
**21.05.2025 Bulletin 2025/21**

(73) Proprietor: **Symrise AG**
**37603 Holzminden Niedersachsen (DE)**

(72) Inventors:
- **NORDZIEKE, Steffen**
**37081 Göttingen (DE)**
- **GENRICH, Florian**
**37586 Dassel (DE)**
- **KRÄLING, Ricarda**
**37603 Holzminden (DE)**
- **WÜNSCH, Matthias**
**37603 Holzminden (DE)**

(74) Representative: **Eisenführ Speiser**
**Patentanwälte Rechtsanwälte PartGmbB**
**Gollierstraße 4**
**80339 München (DE)**

(56) References cited:
**WO-A1-2020/160905**
**US-A1- 2023 149 309**
**US-B2- 11 116 710**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 554 551 B1

## Description

The present invention primarily relates to use of a compound of formula (I) as defined herein as an active ingredient, preferably as a deodorant active. Moreover, it relates to methods for inhibiting or preventing the growth of one or more microorganisms and to a method for inhibiting or reducing the conversion of sweat components to odorous compounds. It also relates to a compound of formula (I) as defined herein for use in the treatment of inflammation, post-inflammatory hyperpigmentation, atopic dermatitis, bacterial vaginosis, tinea pedis, dermatitis exfoliativa neonatorum, impetigo contagiosa, furuncles and carbuncles, folliculitis, impetigo, and/or erysipelas, and for use as an antimicrobial agent, to a mixture comprising one or both compound(s) of formula (I) as defined herein, and to a mixture comprising one or both compound(s) of formula (I) as defined herein and one or more further antimicrobial agent(s). Finally, it relates to a deodorant, rinse-off product, and leave-on product as defined herein.

The present invention is one of a series of inventions related to 3-hydroxypropylesters and structurally related compounds co-filed by the same applicant and on the same date as PCT patent applications, which are titled "3-Hydroxypropylesters as antioxidants and tocopherol boosters", "3-Hydroxypropylesters as solubility enhancers for lipophilic ingredients", "3-Hydroxypropylesters as foam and viscosity modulating agents in surfactant containing formulations", "3-Hydroxypropylesters as emulsifying agents", and "Antimicrobial synergies".

Further aspects of the present invention will arise from the description below, in particular from the examples, as well as from the attached patent claims.

Personal care, pharmaceutical, and household products usually contain antimicrobial agents to increase their shelf-life and to guarantee safety for the consumer's health. The aim is to protect consumers of said products from potentially pathogenic microorganisms that might otherwise grow inside the products, and to stop or delay the biological and/or physicochemical deterioration of the product. The preservation of said products can be achieved by chemical, physical or physicochemical methods. The most common strategy is the incorporation of antimicrobial agents into said products. Common antimicrobial agents are, for example, parabens, formaldehyde releasers, isothiazolinones, organic acids, triclosan, phenoxyethanol and benzyl alcohol. Some antimicrobial agents, however, have the drawback that they might induce undesirable side effects in consumers. These effects might range from mild skin irritation to allergic reactions to the chemicals used. There is thus a large demand for effective antimicrobial agents that avoid the potential drawbacks described above.

The term "microbiome" (from the Greek *micro* meaning "small" and *bíos* meaning "life") was originally defined in 1988 by Whipps *et al.* as "a characteristic microbial community occupying a reasonably well-defined habitat which has distinct physio-chemical properties. The term thus not only refers to the microorganisms involved but also encompasses their theatre of activity". The natural human microbiome is mandatory for our personal well-being. It strengthens our immune system, digests complex food, provides essential vitamins, and is the first barrier against external aggressors. However, some microorganisms of the microbiome are also involved in unpleasant and unwanted but natural functions leading to e.g. bacterial infections, malodour formation from sweat, or oral diseases like biofilm/plaque formation, caries etc. impacting the personal well-being. International Patent application WO-A-2020/160905 discloses the use of a fatty acid ester comprising two or more fatty acid esters, in a cosmetic product for inhibiting growth of micro-organisms on a mammal's skin or mucosa, and/or preserving the cosmetic product against microbial growth, wherein the fatty acid esters are selected from the group consisting of 3-hydroxypropyl caprylate, 3-hydroxypropyl undecylenate and glyceryl monoundecylenate.

Developing new deodorizing actives to fight human sweat malodour is challenging. Cheap high-throughput laboratory screenings on one hand and expensive, time-consuming *in vivo* studies on the other hand leave much room for failure during the developmental process of each new active ingredient. Traditional approaches often ignore the natural diversity of the human skin microbiome, leading to expensive and time-consuming maldevelopments. There is thus also a large demand for antimicrobial agents that, for instance, work well as deodorant actives.

Hence, it was a primary object of the present invention to provide new ingredients that are highly active against a range of common pathogens and, preferably, work well at low concentrations in personal care, pharmaceutical, and household products, and on (human) skin, mucosa, and other surfaces which are contacted with said products. It was a further object of the present invention to provide new combinations of antimicrobial agents that preferably lead to a synergistic improvement of the antimicrobial activity of the resulting mixture. Further objects underlying the present invention follow from the description below and the present patent claims.

According to a first aspect of the present invention, the primary object is achieved by the use of a compound of formula (I) or of a mixture of both compounds of formula (I) or of a composition comprising one or both compound(s) of formula (I)

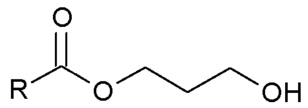

(I)

,

wherein R is selected from the group consisting of n-nonyl and n-undecyl, as an active ingredient, preferably as an antimicrobial agent, more preferably

(i) for inhibiting or preventing the growth of one or more microorganisms on a mammal's, preferably human's, skin or mucosa, preferably skin, more preferably human axillary skin, and/or

(ii) for inhibiting or preventing the growth of one or more microorganisms in a personal care, household, or pharmaceutical product, and/or

(iii) for inhibiting or preventing the growth of one or more microorganisms on a surface,

most preferably as a deodorant active.

The compound of formula (I) is either 3-hydroxypropyldecanoate with the following structure (Ia)

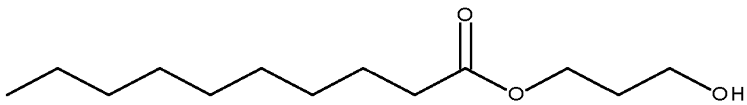

(Ia)

or is 3-hydroxypropyldodecanoate with the following structure (Ib)

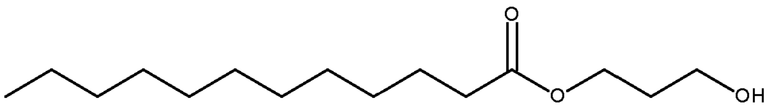

(Ib).

In the context of the studies underlying the present invention, it was surprisingly found that the compounds of formula (I) as defined herein display particularly advantageous antimicrobial properties against various microorganisms commonly found in personal care, pharmaceutical, and household products, in the human microbiome, and on other surfaces. Said products often are in direct contact with the skin, mucosa, or hair of the consumer of the product or with other surfaces used by humans, such as, for instance, kitchen and bathroom surfaces. Hence, it is particularly advantageous to ensure that said products are protected against the growth of potentially pathogenic microorganisms. This ensures that the products are safe to use for the consumer and can be stored for a sufficient amount of time without degrading. More specifically, it was surprisingly found that the compounds of formula (I) as defined herein display particularly advantageous antimicrobial properties against microorganisms which are involved in the production of undesired human sweat odour.

A preferred embodiment of the present invention is the use of a compound of formula (I) or of a mixture of both compounds of formula (I) or of a composition comprising one or both compound(s) of formula (I) as defined herein as an antimicrobial agent, preferably

(i) for inhibiting or preventing the growth of one or more microorganisms in a personal care, household, or pharmaceutical product, and/or

(ii) for inhibiting or preventing the growth of one or more microorganisms on a surface.

According to a preferred embodiment, a compound of formula (I) or a mixture of both compounds of formula (I) or a

composition comprising one or both compound(s) of formula (I) as defined herein is/are used as an ingredient (of a product), preferably an active ingredient, more preferably as a deodorant active, an anti-acne agent, an anti-aging agent, a skin conditioning agent, an anti-dandruff agent, a scalp care agent, a skin care agent, a foot care agent, an intimate care agent, a preservative, and/or a hygiene agent.

Most preferably, a compound of formula (I) or a mixture of both compounds of formula (I) or a composition comprising one or both compound(s) of formula (I) as defined herein is/are used as a deodorant active.

Preferably, the inhibition of the growth of one or more microorganisms (i) on a mammal's, preferably human's, skin or mucosa, preferably skin, more preferably human axillary skin, and/or (ii) in a personal care, household, or pharmaceutical product and/or (iii) on a surface is a reduction of the one or more microorganisms' growth rate and/or a stagnation or reduction of the number of living microorganisms.

Preferably, the one or more microorganisms is/are selected from the group consisting of *Epidermophyton floccosum, Trichophyton rubrum, Staphylococcus epidermidis, Staphylococcus haemolyticus, Staphylococcus hominis, Micrococcus luteus, Acinetobacter haemolyticus, Aspergillus fumigatus, Streptococcus mutans, Porphyromonas gingivalis, Fusobacterium nucleatum, Prevotella intermedia, Malassezia furfur, Malassezia globosa, Malassezia restricta, Malassezia sympodialis, Staphylococcus capitis, Gardnerella vaginalis, Streptococcus agalactiae, Fannyhessea vaginae, Peptoniphilus lacrimalis, Cutibacterium acnes, Staphylococcus aureus, Candida albicans, Aspergillus brasiliensis, Pseudomonas aeruginosa, Escherichia coli, Salmonella enteritidis, Corynebacterium xerosis, Corynebacterium jeikeium, Anaerococcus octavius, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Streptococcus pyogenes, Streptococcus pneumonia, Moraxella osloensis, Kokuria oxytoca, Kokuria pneumonia, Kokuria palustris, Kokuria rhizophilia, Sphingobium yanoikuyae, Stenotrophomonas maltophilia, Pseudomonas cremoricolorata, Bacillus subtilis, Enterobacter gergoviae, Trichophyton mentagrophytes, Brevibacterium epidermidis, Klebsiella pneumonia, Pseudomonas fluorescens, Pseudomonas putida, Penicillium funiculosum, Bacillus licheniformis,* and *Enterococcus hirae.*

According to a preferred embodiment of the use according to the invention, the one or more microorganisms is/are selected from the group consisting of *Epidermophyton floccosum, Trichophyton rubrum, Staphylococcus epidermidis, Staphylococcus haemolyticus, Staphylococcus hominis, Micrococcus luteus, Acinetobacter haemolyticus,* and *Aspergillus fumigatus.*

According to another preferred embodiment of the use according to the invention, the one or more microorganisms is/are selected from the group consisting of *Streptococcus mutans, Porphyromonas gingivalis, Fusobacterium nucleatum,* and *Prevotella intermedia.*

According to another preferred embodiment of the use according to the invention, the one or more microorganisms is/are selected from the group consisting of *Malassezia furfur, Malassezia globosa, Malassezia restricta, Malassezia sympodialis,* and *Staphylococcus capitis.*

According to another preferred embodiment of the use according to the invention, the one or more microorganisms is/are selected from the group consisting of *Gardnerella vaginalis, Streptococcus agalactiae, Lactobacillus acidophilus,* and *Fannyhessea vaginae.*

According to another preferred embodiment of the use according to the invention, the one or more microorganisms is/are selected from the group consisting of *Peptoniphilus lacrimalis, Staphylococcus epidermidis,* and *Cutibacterium acnes.*

According to another preferred embodiment of the use according to the invention, the one or more microorganisms is/are selected from the group consisting of *Peptoniphilus lacrimalis, Staphylococcus epidermidis,* and *Cutibacterium acnes.*

According to another preferred embodiment of the use according to the invention, the one or more microorganisms is/are selected from the group consisting of *Staphylococcus aureus, Candida albicans, Aspergillus brasiliensis, Pseudomonas aeruginosa, Escherichia coli,* and *Salmonella enteritidis.*

According to another preferred embodiment of the use according to the invention, the one or more microorganisms is/are selected from the group consisting of *Staphylococcus hominis, Staphylococcus epidermidis, Corynebacterium xerosis, Corynebacterium jeikeium,* and *Anaerococcus octavius.*

According to another preferred embodiment of the use according to the invention, the one or more microorganisms is/are selected from the group consisting of *Micrococcus luteus, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Escherichia coli, Staphylococcus aureus, Staphylococcus haemolyticus, Streptococcus pyogenes, Streptococcus pneumoniae, Moraxella osloensis, Kokuria oxytoca, Kokuria pneumoniae, Kokuria palustris, Kokuria rhizophilia, Salmonella enteritidis, Sphingobium yanoikuyae, Stenotrophomonas maltophilia, Pseudomonas aeruginosa, Pseudomonas cremoricolorata,* and *Bacillus subtilis.*

Preferably, the one or more microorganisms is/are selected from the group consisting of microorganisms of the

natural microbiota of mammalian skin or mucosa, preferably of mammalian skin, more preferably of human skin, most preferably of human axillary skin.

According to a preferred embodiment of the use according to the invention,

the personal care product is selected from the group consisting of deodorant products, body care and cleansing products, intimate care and cleansing products, foot care and cleansing products, oral care and cleansing products, scalp and hair care and cleansing products, and face care and cleansing products, more preferably the personal care product is selected from the group consisting of deodorant sprays, deodorant aerosols, deodorant roll-ons, deodorant sticks, deodorant creams, foot creams, foot powders, body washes, mouth washes, toothpastes, shampoos, hair conditioner, styling wax, styling gels, styling foams, styling creams, styling mousses, styling serums, styling pomades, micellar waters, intimate washes, cleansers, toners, serums, moisturizers, eye creams, balms, soaps, facial masks, sunscreens, liquids for wet wipes, primers, concealers, foundations, rouges, bronzers, highlighters, eyebrow pencils, eyebrow creams, eyebrow waxes, eyebrow gels, eyebrow powders, eyeshadows, eyeliners, mascara, lipsticks, lip glosses, lip liners, lip balms, face powders, setting powders, setting sprays, and nail polishes,
and/or

the household product is selected from the group consisting of alcohol free glass cleaners, glass cleaners, all-purpose cleaners, all-purpose sprays, degreasers, carpet cleaners, wood cleaners, floor cleaners, laminate cleaners, vinyl cleaners, ceramic cleaners, cork cleaners, linoleum cleaners, porcelain cleaners, stone cleaners, concrete cleaners, parquet cleaners, high gloss floor cleaners, kitchen cleaners, fat solver cleaners, bathroom cleaners, toilet cleaners, chalk cleaners, automatic dish washes, hand dish wash fluids, antibacterial hand dish wash fluids, fabric softeners, fabric care, liquid laundry detergents, powder laundry detergents, and hygiene wash detergents,
and/or

the pharmaceutical product is selected from the group consisting of deodorant sprays, deodorant roll-ons, deodorant sticks, deodorant creams, foot creams, body washes, mouth washes, toothpastes, shampoos, intimate washes, cleansers, serums, moisturizers, eye creams, balms, soaps, facial masks, sunscreens, and liquids for wet wipes.

Preferably, the personal care or pharmaceutical product is a personal care or pharmaceutical product for topical application on a mammal's, preferably human's, skin or mucosa, preferably skin, more preferably human axillary skin.

Preferably, the personal care or pharmaceutical product is selected from the group consisting of creams, ointments, gels, lotions, deodorant sprays, deodorant roll-ons, deodorant sticks, deodorant creams, foot creams, body washes, mouth washes, toothpastes, shampoos, hair conditioner, styling wax, styling gels, styling foams, styling creams, styling mousses, styling serums, styling pomades, micellar waters, intimate washes, cleansers, toners, serums, moisturizers, eye creams, balms, soaps, facial masks, sunscreens, liquids for wet wipes, primers, concealers, foundations, rouges, bronzers, highlighters, eyebrow pencils, eyebrow creams, eyebrow waxes, eyebrow gels, eyebrow powders, eyeshadows, eyeliners, mascara, lipsticks, lip glosses, lip liners, lip balms, face powders, setting powders, setting sprays, and nail polishes.

According to another preferred embodiment of the use according to the invention, the surface is a solid surface, preferably selected from the group consisting of kitchen surfaces, bathroom surfaces, drums of washing machines, cutlery, crockery, wood flooring (solid wood flooring, engineered wood flooring), laminate, vinyl, ceramic, cork, linoleum, porcelain, stone, concrete, and parquet, or is a non-solid surface, preferably selected from the group consisting of woven materials and leathers, more preferably fabrics, carpets, tissues, clothes, sheets, and sponges.

Another aspect of the present invention relates to a method for inhibiting or preventing the growth of one or more microorganisms, comprising or consisting of the following steps:

(a) Providing a compound of formula (I) or a mixture of both compounds of formula (I) or a composition or product comprising one or both compound(s) of formula (I) as defined herein, and

(b) bringing the compound or mixture or composition or product provided in step (a) in contact with one or more microorganisms.

Another aspect of the present invention relates to a method

(i) for inhibiting or preventing the growth of one or more microorganisms on a mammal's, preferably human's, skin or mucosa, preferably skin, more preferably human axillary skin, and/or

(ii) for inhibiting or preventing the growth of one or more microorganisms in a personal care, household, or

pharmaceutical product, and/or

(iii) for inhibiting or preventing the growth of one or more microorganisms on/in a surface,

comprising or consisting of the following steps:

(a) Providing a compound of formula (I) or a mixture of both compounds of formula (I) or a composition or product comprising one or both compound(s) of formula (I) as defined herein, and

(b) bringing the compound or mixture or composition or product provided in step (a)

(i) in contact with a mammal's, preferably human's, skin or mucosa, preferably skin, more preferably human axillary skin, on which the growth of one or more microorganisms is to be inhibited or prevented, and/or

(ii) in contact with the other ingredients of a personal care, household, or pharmaceutical product, in which the growth of one or more microorganisms is to be inhibited or prevented, and/or

(iii) in contact with a surface, on/in which the growth of one or more microorganisms is to be inhibited or prevented.

A preferred embodiment of the present invention relates to a method

(i) for inhibiting or preventing the growth of one or more microorganisms in a personal care, household, or pharmaceutical product, and/or

(ii) for inhibiting or preventing the growth of one or more microorganisms on/in a surface,

comprising or consisting of the following steps:

(a) Providing a compound of formula (I) or a mixture of both compounds of formula (I) or a composition or product comprising one or both compound(s) of formula (I) as defined herein, and

(b) bringing the compound or mixture or composition or product provided in step (a)

(i) in contact with the other ingredients of a personal care, household, or pharmaceutical product, in which the growth of one or more microorganisms is to be inhibited or prevented, and/or

(ii) in contact with a surface, on/in which the growth of one or more microorganisms is to be inhibited or prevented.

Preferably, the one or more microorganisms, whose growth is inhibited or prevented, is/are selected from the group consisting of *Epidermophyton floccosum, Trichophyton rubrum, Staphylococcus epidermidis, Staphylococcus haemolyticus, Staphylococcus hominis, Micrococcus luteus, Acinetobacter haemolyticus, Aspergillus fumigatus, Streptococcus mutans, Porphyromonas gingivalis, Fusobacterium nucleatum, Prevotella intermedia, Malassezia furfur, Malassezia globosa, Malassezia restricta, Malassezia sympodialis, Staphylococcus capitis, Gardnerella vaginalis, Streptococcus agalactiae, Fannyhessea vaginae, Peptoniphilus lacrimalis, Cutibacterium acnes, Staphylococcus aureus, Candida albicans, Aspergillus brasiliensis, Pseudomonas aeruginosa, Escherichia coli, Salmonella enteritidis, Corynebacterium xerosis, Corynebacterium jeikeium, Anaerococcus octavius, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Streptococcus pyogenes, Streptococcus pneumonia, Moraxella osloensis, Kokuria oxytoca, Kokuria pneumonia, Kokuria palustris, Kokuria rhizophilia, Sphingobium yanoikuyae, Stenotrophomonas maltophilia, Pseudomonas cremoricolorata, Bacillus subtilis, Enterobacter gergoviae, Trichophyton mentagrophytes, Brevibacterium epidermidis, Klebsiella pneumonia, Pseudomonas fluorescens, Pseudomonas putida, Penicillium funiculosum, Bacillus licheniformis,* and *Enterococcus hirae.*

According to a preferred embodiment of the method according to the invention, the one or more microorganisms, whose growth is inhibited or prevented, is/are selected from the group consisting of *Epidermophyton floccosum, Trichophyton rubrum, Staphylococcus epidermidis, Staphylococcus haemolyticus, Staphylococcus hominis, Micrococcus luteus, Acinetobacter haemolyticus,* and *Aspergillus fumigatus.*

According to another preferred embodiment of the method according to the invention, the one or more microorganisms, whose growth is inhibited or prevented, is/are selected from the group consisting of *Streptococcus*

*mutans, Porphyromonas gingivalis, Fusobacterium nucleatum,* and *Prevotella intermedia.*

According to another preferred embodiment of the method according to the invention, the one or more microorganisms, whose growth is inhibited or prevented, is/are selected from the group consisting of *Malassezia furfur, Malassezia globosa, Malassezia restricta, Malassezia sympodialis,* and *Staphylococcus capitis.*

According to another preferred embodiment of the method according to the invention, the one or more microorganisms, whose growth is inhibited or prevented, is/are selected from the group consisting of *Gardnerella vaginalis, Streptococcus agalactiae, Lactobacillus acidophilus,* and *Fannyhessea vaginae.*

According to another preferred embodiment of the method according to the invention, the one or more microorganisms, whose growth is inhibited or prevented, is/are selected from the group consisting of *Peptoniphilus lacrimalis, Staphylococcus epidermidis,* and *Cutibacterium acnes.*

According to another preferred embodiment of the method according to the invention, the one or more microorganisms, whose growth is inhibited or prevented, is/are selected from the group consisting of *Peptoniphilus lacrimalis, Staphylococcus epidermidis,* and *Cutibacterium acnes.*

According to another preferred embodiment of the method according to the invention, the one or more microorganisms, whose growth is inhibited or prevented, is/are selected from the group consisting of *Staphylococcus aureus, Candida albicans, Aspergillus brasiliensis, Pseudomonas aeruginosa, Escherichia coli,* and *Salmonella enteritidis.*

According to another preferred embodiment of the method according to the invention, the one or more microorganisms, whose growth is inhibited or prevented, is/are selected from the group consisting of *Staphylococcus hominis, Staphylococcus epidermidis, Corynebacterium xerosis, Corynebacterium jeikeium,* and *Anaerococcus octavius.*

According to another preferred embodiment of the method according to the invention, the one or more microorganisms, whose growth is inhibited or prevented, is/are selected from the group consisting of *Micrococcus luteus, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Escherichia coli, Staphylococcus aureus, Staphylococcus haemolyticus, Streptococcus pyogenes, Streptococcus pneumoniae, Moraxella osloensis, Kokuria oxytoca, Kokuria pneumoniae, Kokuria palustris, Kokuria rhizophilia, Salmonella enteritidis, Sphingobium yanoikuyae, Stenotrophomonas maltophilia, Pseudomonas aeruginosa, Pseudomonas cremoricolorata,* and *Bacillus subtilis.*

Preferably, the inhibition of the growth of one or more microorganisms (i) on a mammal's, preferably human's, skin or mucosa, preferably skin, more preferably human axillary skin, and/or (ii) in a personal care, household, or pharmaceutical product and/or (iii) on a surface is a reduction of the one or more microorganisms' growth rate and/or a stagnation or reduction of the number of living microorganisms.

According to a preferred embodiment of the method according to the invention,

the personal care product is selected from the group consisting of deodorant products, body care and cleansing products, intimate care and cleansing products, foot care and cleansing products, oral care and cleansing products, scalp care and cleansing products, and face care and cleansing products, more preferably the personal care product is selected from the group consisting of deodorant sprays, deodorant roll-ons, deodorant sticks, deodorant creams, foot creams, body washes, mouth washes, toothpastes, shampoos, hair conditioner, styling wax, styling gels, styling foams, styling creams, styling mousses, styling serums, styling pomades, micellar waters, intimate washes, cleansers, toners, serums, moisturizers, eye creams, balms, soaps, facial masks, sunscreens, liquids for wet wipes, primers, concealers, foundations, rouges, bronzers, highlighters, eyebrow pencils, eyebrow creams, eyebrow waxes, eyebrow gels, eyebrow powders, eyeshadows, eyeliners, mascara, lipsticks, lip glosses, lip liners, lip balms, face powders, setting powders, setting sprays, and nail polishes,
and/or

the household product is selected from the group consisting of alcohol free glass cleaners, glass cleaners, all-purpose cleaners, all-purpose sprays, degreasers, carpet cleaners, wood cleaners, floor cleaners, laminate cleaners, vinyl cleaners, ceramic cleaners, cork cleaners, linoleum cleaners, porcelain cleaners, stone cleaners, concrete cleaners, parquet cleaners, high gloss floor cleaners, kitchen cleaners, fat solver cleaners, bathroom cleaners, toilet cleaners, chalk cleaners, automatic dish washes, hand dish wash fluids, antibacterial hand dish wash fluids, fabric softeners, fabric care, liquid laundry detergents, powder laundry detergents, and hygiene wash detergents,
and/or

the pharmaceutical product is selected from the group consisting of deodorant sprays, deodorant roll-ons, deodorant sticks, deodorant creams, foot creams, body washes, mouth washes, toothpastes, shampoos, intimate washes, cleansers, serums, moisturizers, eye creams, balms, soaps, facial masks, sunscreens, and liquids for wet wipes.

According to another preferred embodiment of the method according to the invention, the personal care or pharmaceutical product is a personal care or pharmaceutical product for topical application on a mammal's, preferably human's, skin or mucosa, preferably skin, more preferably human axillary skin.

Preferably, the personal care or pharmaceutical product is selected from the group consisting of creams, ointments, gels, lotions, deodorant sprays, deodorant roll-ons, deodorant sticks, deodorant creams, foot creams, body washes, mouth washes, toothpastes, shampoos, hair conditioner, styling wax, styling gels, styling foams, styling creams, styling mousses, styling serums, styling pomades, micellar waters, intimate washes, cleansers, toners, serums, moisturizers, eye creams, balms, soaps, facial masks, sunscreens, liquids for wet wipes, primers, concealers, foundations, rouges, bronzers, highlighters, eyebrow pencils, eyebrow creams, eyebrow waxes, eyebrow gels, eyebrow powders, eyeshadows, eyeliners, mascara, lipsticks, lip glosses, lip liners, lip balms, face powders, setting powders, setting sprays, and nail polishes.

According to another preferred embodiment of the method according to the invention, the surface is a solid surface, preferably selected from the group consisting of kitchen surfaces, bathroom surfaces, drums of washing machines, cutlery, crockery, wood flooring (solid wood flooring, engineered wood flooring), laminate, vinyl, ceramic, cork, linoleum, porcelain, stone, concrete, and parquet, or is a non-solid surface, preferably selected from the group consisting of woven materials and leathers, preferably fabrics, carpets, tissues, clothes, sheets, and sponges. By bringing said surfaces in contact with a compound of formula (I) or a mixture of both compounds of formula (I) or a composition or product comprising one or both compound(s) of formula (I) as defined herein, the growth of one or more microorganisms on/in said surfaces can advantageously be inhibited or prevented.

Another aspect of the present invention relates to the (cosmetic) use of a compound of formula (I) or of a mixture of both compounds of formula (I) or of a composition or product comprising one or both compound(s) of formula (I) as defined herein for inhibiting or reducing the conversion of sweat components to odorous compounds, preferably for inhibiting or reducing the conversion of 5-amino-2-[[(E)-3-methylhex-2-enoyl]amino]-5-oxo-pentanoic acid to 3-methylhex-2-enoic acid and/or the conversion of 5-amino-2-(3-methylenehexanoylamino)-5-oxo-pentanoic acid to 3-hydroxy-3-methylhexanoic acid, preferably on a mammal's, preferably human's, skin or mucosa, preferably skin, more preferably on human axillary skin.

Another aspect of the present invention relates to a (cosmetic) method for inhibiting or reducing the conversion of one or more sweat component(s) to one or more odorous compound(s), preferably wherein the odorous compound(s) is/are selected from the group consisting of acetic acid, propanoic acid, isobutyric acid, butyric acid, 2-methylbutyric acid, valeric acid, capronic acid, 3-mercapto-3-methyl-hexanol, 3-methyl-2-hexenoic acid, biphenyl, diphenyl oxide, caprylic acid, pelargonic acid, capric acid, indole, lauric acid, 3-phenylpropanoic acid, myristic acid, pentadecanoic acid, palmitic acid, cis-9-hexadecenoic acid, stearic acid, 3-methyl 3-hexenoic acid, 3-methyl 3-hydroxy hexanoic acid, 3-methyl 3-sulfanylhexanol, isovaleric acid, 5α-androst-16-en-3-one, 3-methylhex-2-enoic acid, and 3-hydroxy-3-methyl-hexanoic acid, more preferably for inhibiting or reducing the conversion of 5-amino-2-[[(E)-3-methylhex-2-enoyl]amino]-5-oxo-pentanoic acid to 3-methylhex-2-enoic acid and/or for inhibiting or reducing the conversion of 5-amino-2-(3-methylenehexanoylamino)-5-oxo-pentanoic acid to 3-hydroxy-3-methyl-hexanoic acid,
preferably on a mammal's, preferably human's, skin or mucosa, more preferably skin, most preferably on human axillary skin, comprising or consisting of the following steps:

(a) Providing a compound of formula (I) or a mixture of both compounds of formula (I) or a composition or product comprising one or both compound(s) of formula (I) as defined herein, and

(b) bringing the compound or mixture or composition or product provided in step (a) in contact with one or more microorganisms, preferably gram positive bacteria, more preferably selected from the genera *Staphylococcus, Corynebacterium,* and *Anaerococcus,* most preferably selected from the group consisting of *Staphylococcus hominis, Staphylococcus epidermidis, Corynebacterium xerosis, Corynebacterium jeikeium,* and *Anaerococcus octavius,* preferably on a mammal's, preferably human's, skin or mucosa, more preferably skin, most preferably on human axillary skin.

During the studies underlying the present invention, a new model system based on the human sweat microbiome, mimicking natural sweat odour development was successfully developed. Combining this model with the applicant's in-house expertise for the screening of deodorant antimicrobials surprisingly revealed one aspect of the present invention, i.e. that the compounds of formula (I) as defined herein can be used as new sustainable deodorant actives. Applying the *ex vivo* sweat model for the compounds of formula (I) as defined herein, further advantageous features of said compounds were discovered: When applied directly to fresh, odourless sweat, it can reduce the sweat odour development over the course of 48h in a concentration-dependent manner. Sweat evaluation values were reduced around 1.5 compared to untreated samples at 24h and 0.6 at 48h, respectively. Simultaneously, the addition the compounds of formula (I) as defined herein for instance reduces the conversion of non-odorous, glycine-coupled

precursors to volatile and perceivable end products and limits the production of small organic acids, most prominently acetic acid, propanoic acid, isobutyric acid, 2-methylbutyric acid, and indole. Through analysis of the microbial cell counts and 16S RNA gene sequencing, an inhibiting effect on *Anaerococcus* was discovered.

Another aspect of the present invention relates to a compound of formula (I) or a mixture of both compounds of formula (I) or a composition or product comprising one or both compound(s) of formula (I) as defined herein for use in the treatment of inflammation, preferably skin inflammation, post-inflammatory hyperpigmentation, atopic dermatitis, bacterial vaginosis, tinea pedis, dermatitis exfoliativa neonatorum, impetigo contagiosa, furuncles and carbuncles, folliculitis, impetigo, and/or erysipelas.

Another aspect of the present invention relates to a compound of formula (I) or a mixture of both compounds of formula (I) or a composition or product comprising one or both compound(s) of formula (I) as defined herein for use as an antimicrobial agent, preferably for inhibiting or preventing the growth of one or more microorganisms on a mammal's, preferably human's, skin or mucosa, preferably skin, more preferably human axillary skin.

The present invention further relates to a product for use as defined herein, wherein the product is a pharmaceutical product for topical application on a mammal's, preferably human's, skin or mucosa, preferably skin, preferably selected from the group consisting of creams, ointments, gels, lotions, more preferably deodorant sprays, deodorant roll-ons, deodorant sticks, deodorant creams, foot creams, body washes, mouth washes, toothpastes, shampoos, intimate washes, cleansers, serums, moisturizers, eye creams, balms, soaps, facial masks, sunscreens, and liquids for wet wipes.

Another aspect of the present invention relates to a mixture, preferably a personal care, household, or pharmaceutical product, comprising one or both compound(s) of formula (I) or a mixture of both compounds of formula (I) or a composition comprising one or both compound(s) of formula (I) as defined herein, preferably comprising a total amount of compound(s) of formula (I) as defined herein of from 0.01 to 10 wt.%, preferably of 0.05 to 2.5 wt.%, more preferably of 0.1 to 1 wt.%, based on the total weight of the mixture or product.

Another aspect of the present invention relates to a mixture, preferably as defined herein, comprising or consisting of

(i) one or both compound(s) of formula (I) as defined herein, and

(ii) one or more further antimicrobial agent(s) (that are different from the compound(s) of formula (I) as defined herein),

preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i),

more preferably wherein the one or more further antimicrobial agent(s) of constituent (ii) is/are

(ii-1) selected from the group consisting of 1,2-decanediol (Decylene Glycol), 1,2-heptanediol, 1,2-hexanediol, 1,2-nonanediol, 1,2-octanediol, 1,2-pentanediol, 1,2-propanediol, 1,3-propanediol, 2,3-decanediol, 2,3-heptanediol, 2,3-hexanediol, 2,3-nonanediol, 2,3-octanediol, 2,3-pentanediol, 2-benzylheptanol, 2-hydroxyacetophenone, 2-methyl 5-cyclohexylpentanol, 3-hydroxypropyl 2-hydroxybenzoate, 4-hydroxyacetophenone, alkyl (C 12-22) trimethyl ammonium bromide and chloride, anisic acid 3-hydroxypropylester, anisic acid and its salts, ascorbic acid and salts thereof, ascorbyl palmitate , azelaic acid, azeloyl diglycinate, benzalkonium bromide, benzalkonium chloride, benzalkonium saccharinate, benzethonium chloride, benzoic acid, benzoic acid 3-hyroxypropylester, benzyl alcohol, benzyl salicylate, benzysothiazolinone, bisabolol, bronopol (2-bromo-2-nitropropane-1,3-diol), butylene glycol, butylparaben, camphor, caprylhydroxamic acid, capryloyl glycine, capryloyl/caproyl anhydro methyl glucamide, caprylyl glyceryl ether, caprylyl/capric triglyceride, cetylpyridinium chloride, chlorhexidine, chlorhexidine diacetate, chlorhexidine digluconate, chlorhexidine dihydrochloride, chlorocresol, chloroxylenol, chlorphenesin, citronellyl methylcrotonate, climbazole, cyclohexylglycerin, dehydroacetic acid, diazolidinyl urea, dimethyl phenylbutanol, dimethyl phenylpropanol, dipropylene glycol, disodium EDTA, DMDM hydantoin, ethanol, ethyl lauroyl arginate HCl, ethyl lauroyl arginate laurate, ethylhexylglycerin, ethylparaben, eucalyptol, farnesol, garcinia mangostana peel extract, gluconic acid and salts thereof, glycerine, glyceryl caprate, glyceryl caprylate, glyceryl heptanoate, glyceryl laurate, glyceryl undecylenate, glycolipids, heptylglycerin, hexamidine, hexamidine diisethionate, hexylene glycol, hexylglycerin, hydroxyethoxyphenyl butanol, hydroxyethoxyphenyl butanone, imidazolidinyl urea, iodopropynyl butylcarbamate, isoamyl laurate, isobutylparaben, isopropylparaben, itaconic acid and salts thereof, jasmol, lactic acid, lauryl alcohol, levulinic acid, mandelic acid, mannitol, menthol, methyl salicylate, methyl undecylenate, methylbenzyl alcohol, methylchloroisothiazolinone, methylheptylglycerin, methylisothiazolinone, methylparaben and salts thereof, methylpropanediol, morinda citrifolia callus culture lysate, octenidine HCl, octylisothiazolinone, o-cymen-5-ol , panthenol, PCA ethyl cocoyl arginate, phenethyl alcohol, phenoxyethanol, phenylpropanol, piroctone olamine, polyarginine, polyglyceryl-10 laurate, polyglyceryl-2 caprate, polyglyceryl-3 caprylate, polyglyceryl-4 caprylate/caprate, polyglyceryl-4 laurate/sebacate, polylysine, potassium sorbate, propanediol caprylate, propanediol undecylenate, propylene glycol, propylene glycol caprylate, propylparaben, raspberry ketone, saccharide isomerate, salicylic acid, salvia officinalis (sage) oil, silver chloride, silver citrate, sodium anisate, sodium benzoate, sodium caproyl lactylate, sodium caproyl/lauroyl lactylate, sodium ethylparaben, sodium lactate, sodium lauroyl lactylate, sodium levulinate, levulinic acid, sodium methylparaben, sodium phytate, phytic acid, sodium propylparaben, sorbic acid, sorbitan caprylate, sorbitol, TEA-salicylate, tetraselmis extract, tetrasodium glutamate diacetate, thymol, triclocarban, triclosan, triethyl citrate, trisodium dicarboxymethyl alaninate, trisodium ethylenediamine disuccinate, tropolone, undecylenic acid, undecylenoyl glycine, xylityl caprylate, xylityl sesquicaprylate, zinc citrate, zinc lactate, zinc neodecanoate, zinc pyrithione, zinc ricinoleate, zinc undecylenate and capryloyl/caproyl anhydro methyl glucamide (and) water.

According to another preferred embodiment, the one or more further antimicrobial agent(s) of constituent (ii) of the mixture is/are

(ii-2) selected from the group consisting of 1,2-decanediol (decylene glycol), 1,2-heptanediol, 1,2-hexanediol, 1,2-nonanediol, 1,2-octanediol, 1,2-pentanediol, 1,3-propanediol, 2,3-heptanediol, 2,3-hexanediol, 2,3-nonanediol, 2,3-octanediol, 2,3-pentanediol, 2-benzylheptanol, 2-methyl 5-cyclohexylpentanol, 4-hydroxyacetophenone, anisic acid 3-hydroxypropylester, anisic acid and salts thereof, ascorbic acid and salts thereof, benzalkonium chloride, benzethonium chloride, benzoic acid, benzoic acid 3-hyroxypropylester, benzyl alcohol, benzyl salicylate, bisabolol, butylene glycol, caprylhydroxamic acid, capryloyl glycine, caprylyl glyceryl ether, cetylpyridinium chloride, chlorphenesin, citronellyl methylcrotonate, climbazole, cyclohexylglycerin, dehydroacetic acid, dimethyl phenylbutanol, dimethyl phenylpropanol, dipropylene glycol, disodium EDTA, ethanol, ethyl lauroyl arginate HCl, ethyl lauroyl arginate laurate, ethylhexylglycerin, ethylparaben, farnesol, garcinia mangostana peel extract, gluconic acid and salts thereof, glycerine, glyceryl caprate, glyceryl caprylate, glyceryl laurate, glyceryl undecylenate, heptylglycerin, hexamidine diisethionate, hexylene glycol, hexylglycerin, hydroxyethoxyphenyl butanol, hydroxyethoxyphenyl butanone, imidazolidinyl urea, isobutylparaben, itaconic acid and salts thereof, jasmol, lactic acid, lauryl alcohol, levulinic acid, mandelic acid, methylbenzyl alcohol, methylheptylglycerin, methylparaben and salts thereof, morinda citrifolia callus culture lysate, octenidine HCl, o-cymen-5-ol, panthenol, PCA ethyl cocoyl arginate, phenethyl alcohol, phenoxyethanol, phenylpropanol, piroctone olamine, polyglyceryl-10 laurate, polyglyceryl-2 caprate, polyglyceryl-3 caprylate, polylysine, potassium sorbate, propanediol caprylate, propanediol undecylenate, propylene glycol, propylene glycol caprylate, propylparaben, raspberry ketone, salicylic acid, salvia officinalis (sage) oil, sodium benzoate, sodium caproyl lactylate, sodium caproyl/lauroyl lactylate, sodium lactate, sodium lauroyl lactylate, sodium levulinate, levulinic acid, sodium phytate, phytic acid, sorbic acid, sorbitan caprylate, sorbitol, tetraselmis extract, tetrasodium glutamate diacetate, thymol, triethyl citrate, trisodium dicarboxymethyl alaninate, trisodium ethylenediamine disuccinate, tropolone, undecylenoyl glycine, xylityl caprylate, xylityl sesquicaprylate, zinc citrate, zinc lactate, zinc neodecanoate, zinc ricinoleate, and zinc undecylenate,

more preferably is/are

(ii-3) selected from the group consisting of 1,2-decanediol (decylene glycol), 1,2-heptanediol, 1,2-hexanediol, 1,2-nonanediol, 1,2-octanediol, 1,2-pentanediol, 1,3-propanediol, 2,3-heptanediol, 2,3-hexanediol, 2,3-nonanediol, 2,3-octanediol, 2,3-pentanediol, 2-methyl 5-cyclohexylpentanol, 4-hydroxyacetophenone, anisic acid 3-hydroxypropylester, anisic acid and salts thereof, benzoic acid, benzoic acid 3-hyroxypropylester, benzyl alcohol, benzyl salicylate, bisabolol, butylene glycol, caprylhydroxamic acid, capryloyl glycine, caprylyl glyceryl ether, chlorphenesin, citronellyl methylcrotonate, climbazole, cyclohexylglycerin, dehydroacetic acid, dimethyl phenylbutanol, dimethyl phenylpropanol, dipropylene glycol, ethanol, ethyl lauroyl arginate HCl, ethyl lauroyl arginate laurate, ethylhexylglycerin, ethylparaben, farnesol, garcinia mangostana peel extract, glycerine, glyceryl caprate, glyceryl caprylate, glyceryl laurate, glyceryl undecylenate, heptylglycerin, hexylene glycol, hydroxyethoxyphenyl butanol, hydroxyethoxyphenyl butanone, itaconic acid and salts thereof, lactic acid, lauryl alcohol, levulinic acid, methylhepthylglycerin, methylparaben and salts thereof, morinda citrifolia callus culture lysate, octenidine HCl, o-cymen-5-ol, panthenol, phenoxyethanol, phenylpropanol, piroctone olamine, polyglyceryl-2 caprate, polyglyceryl-3 caprylate, potassium sorbate, propanediol caprylate, propanediol undecylenate, propylene glycol, propylene glycol caprylate, raspberry ketone, salvia officinalis (sage) oil, sodium benzoate, sodium caproyl/lauroyl lactylate, sodium levulinate, levulinic acid, sorbic acid, sorbitan caprylate, sorbitol, tetraselmis extract, tetrasodium glutamate diacetate, triethyl citrate, trisodium ethylenediamine disuccinate, tropolone, undecylenoyl glycine, xylityl caprylate, xylityl sesquicaprylate, zinc citrate, zinc lactate, zinc neodecanoate, zinc ricinoleate, and zinc undecylenate,

more preferably is/are

(ii-4) selected from the group consisting of 1,2-decanediol (decylene glycol), 1,2-heptanediol, 1,2-hexanediol, 1,2-nonanediol, 1,2-octanediol, 1,2-pentanediol (pentylene glycol), 1,3-propanediol, 2-methyl 5-cyclohexylpentanol, 4-hydroxyacetophenone, benzoic acid 3-hyroxypropylester, benzyl alcohol, benzyl salicylate, bisabolol, butylene glycol, caprylhydroxamic acid, capryloyl glycine, caprylyl glyceryl ether, chlorphenesin, citronellyl methylcrotonate, dimethyl phenylbutanol, dimethyl phenylpropanol, dipropylene glycol, ethylhexylglycerin, ethylparaben, farnesol, garcinia mangostana peel extract, glyceryl caprylate, glyceryl laurate, glyceryl undecylenate, heptylglycerin, hexylene glycol, hydroxyethoxyphenyl butanol, hydroxyethoxyphenyl butanone, itaconic acid and salts thereof, lauryl alcohol, methylhepthylglycerin, methylparaben and salts thereof, morinda citrifolia callus culture lysate, octenidine HCl, o-cymen-5-ol, panthenol, phenoxyethanol, phenylpropanol, piroctone olamine, polyglyceryl-2 caprate, polyglyceryl-3 caprylate, potassium sorbate, propanediol caprylate, propanediol undecylenate, propylene glycol, propylene glycol caprylate, raspberry ketone, salvia officinalis (sage) oil, sodium benzoate, sodium caproyl/lauroyl lactylate, triethyl citrate, undecylenoyl glycine, xylityl caprylate, xylityl sesquicaprylate, zinc citrate, zinc lactate, zinc neodecanoate, zinc ricinoleate, and zinc undecylenate,

most preferably is/are

(ii-5) selected from the group consisting of 1,2-decanediol (decylene glycol), 1,2-heptanediol, 1,2-hexanediol, 1,2-nonanediol, 1,2-octanediol, 1,2-pentanediol (pentylene glycol), 1,3-propanediol, benzyl salicylate, bisabolol, caprylhydroxamic acid, chlorphenesin, ethylhexylglycerin, ethylparaben, glyceryl caprylate, hydroxyethoxyphenyl butanol, hydroxyethoxyphenyl butanone, itaconic acid and salts thereof, methylhepthylglycerin, methylparaben and salts thereof, o-cymen-5-ol, phenoxyethanol, potassium sorbate, raspberry ketone, and sodium benzoate.

According to a preferred embodiment, the mixture according to the invention comprises or consists of

(i)

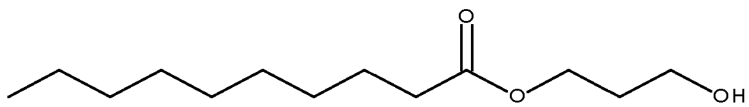

(3-hydroxypropyldecanoate, compound of structure (Ia))
and
(ii) 1,2-decanediol,

preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) 1,2-heptanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) 1,2-hexanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) 1,2-nonanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) 1,2-octanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) 1,2-pentanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) 1,3-propanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) 2-methyl 5-cyclohexylpentanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) 4-hydroxyacetophenone, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) benzoic acid 3-hyroxypropylester, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) benzyl alcohol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) benzyl salicylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) bisabolol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) butylene glycol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) caprylhydroxamic acid, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) capryloyl glycine, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) caprylyl glyceryl ether, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) chlorphenesin, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) citronellyl methylcrotonate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) dimethyl phenylbutanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) dimethyl phenylpropanol, preferably wherein the total amounts of constituents (i) and (ii), respectively, contained in the mixture are such that constituent (i) synergistically improves the antimicrobial effect of

constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) dipropylene glycol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) ethylhexylglycerin, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) ethylparaben, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) farnesol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) garcinia mangostana peel extract, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) glyceryl caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) glyceryl laurate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) glyceryl undecylenate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) heptylglycerin, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) hexylene glycol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) hydroxyethoxyphenyl butanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) hydroxyethoxyphenyl butanone, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) itaconic acid, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) one or more salt(s) of itaconic acid, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) lauryl alcohol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) methylhepthylglycerin, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) methylparaben, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) one or more salt(s) of methylparaben, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) morinda citrifolia callus culture lysate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) octenidine HCl, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) o-cymen-5-ol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) panthenol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) phenoxyethanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) phenylpropanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) piroctone olamine, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) polyglyceryl-2 caprate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) polyglyceryl-3 caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) potassium sorbate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) propanediol caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) propanediol undecylenate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) propylene glycol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) propylene glycol caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) raspberry ketone, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) salvia officinalis (sage) oil, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) sodium benzoate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) sodium caproyl/lauroyl lactylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) triethyl citrate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) undecylenoyl glycine, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) xylityl caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) xylityl sesquicaprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) zinc citrate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) zinc lactate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) zinc neodecanoate, preferably wherein the total amounts of constituents (i) and (ii), respectively, contained in the mixture are such that constituent (i) synergistically improves the antimicrobial effect of

constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) zinc ricinoleate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldecanoate and (ii) zinc undecylenate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to a preferred embodiment, the mixture according to the invention comprises or consists of

(i)

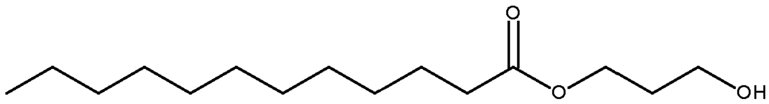

(3-hydroxypropyldodecanoate, compound of structure (Ib))
and

(ii) 1,2-decanediol,

preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) 1,2-heptanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) 1,2-hexanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) 1,2-nonanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) 1,2-octanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) 1,2-pentanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) 1,3-propanediol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) 2-methyl 5-cyclohexylpentanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) 4-hydroxyacetophenone, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) benzoic acid 3-hyroxypropylester, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) benzyl alcohol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) benzyl salicylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) bisabolol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) butylene glycol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) caprylhydroxamic acid, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) capryloyl glycine, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) caprylyl glyceryl ether, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) chlorphenesin, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) citronellyl methylcrotonate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) dimethyl phenylbutanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) dimethyl phenylpropanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) dipropylene glycol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) ethylhexylglycerin, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) ethylparaben, preferably wherein the total amounts of constituents (i) and (ii), respectively, contained in the mixture are such that constituent (i) synergistically improves the antimicrobial effect of

constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) farnesol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) garcinia mangostana peel extract, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) glyceryl caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) glyceryl laurate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) glyceryl undecylenate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) heptylglycerin, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) hexylene glycol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) hydroxyethoxyphenyl butanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) hydroxyethoxyphenyl butanone, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) itaconic acid, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) one or more salt(s) of itaconic acid, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) lauryl alcohol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) methylhepthylglycerin, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) methylparaben, preferably wherein the total amounts of constituents (i) and (ii), respectively, contained in the mixture are such that constituent (i) synergistically improves the antimicrobial effect of

constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) one or more salt(s) of methylparaben, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) morinda citrifolia callus culture lysate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) octenidine HCl, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) o-cymen-5-ol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) panthenol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) phenoxyethanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) phenylpropanol, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) piroctone olamine, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) polyglyceryl-2 caprate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) polyglyceryl-3 caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) potassium sorbate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) propanediol caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) propanediol undecylenate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) propylene glycol, preferably wherein the total amounts of constituents (i) and (ii), respectively, contained in the mixture are such that constituent (i) synergistically improves the antimicrobial effect of

constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) propylene glycol caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) raspberry ketone, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) salvia officinalis (sage) oil, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) sodium benzoate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) sodium caproyl/lauroyl lactylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) triethyl citrate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) undecylenoyl glycine, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) xylityl caprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) xylityl sesquicaprylate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) zinc citrate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) zinc lactate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) zinc neodecanoate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) zinc ricinoleate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

According to another preferred embodiment, the mixture according to the invention comprises or consists of (i) 3-hydroxypropyldodecanoate and (ii) zinc undecylenate, preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i).

Another aspect of the present invention relates to a deodorant comprising (or consisting of) one or both

compound(s) of formula (I) as defined herein.

Preferably, the deodorant is in the form of a roll-on, a pump spray, a stick, an aerosol spray, or a cream.

Another aspect of the present invention relates to a rinse-off, preferably cosmetic or pharmaceutical, product comprising (or consisting of) one or both compound(s) of formula (I) as defined herein.

Preferably, the rinse-off product is a solid, a liquid, a foam, a paste, a gum, a gel, or a concentrate, more preferably in the form of a shampoo, a chewing gum, a soap, a conditioner, a mouthwash, a toothpaste, a body wash, a handwash, or an intimate gel, most preferably in the form of a solid shampoo, a micellar water, a liquid shampoo, a solid body wash, a liquid body wash, a solid soap, a liquid soap, a solid conditioner, or a liquid conditioner.

Another aspect of the present invention relates to a leave-on, preferably cosmetic or pharmaceutical, product comprising (or consisting of) one or both compound(s) of formula (I) as defined herein.

Preferably, the leave-on product is in the form of an emulsion, more preferably in the form of a cream, an ointment, a foam, a lotion, a gel, a paste, a stick, or a powder.

(Preferred) embodiments of the uses according to the invention correspond to or can be derived from the (preferred) embodiments of the methods according to the invention which are explained above or *vice versa.* (Preferred) embodiments of the uses according to the invention correspond to or can be derived from the (preferred) embodiments of the mixtures according to the invention which are explained above or *vice versa.* (Preferred) embodiments of the uses according to the invention correspond to or can be derived from the (preferred) embodiments of the products or compositions according to the invention which are explained above or *vice versa.* (Preferred) embodiments of the methods according to the invention correspond to or can be derived from the (preferred) embodiments of the mixtures according to the invention which are explained above or *vice versa.* (Preferred) embodiments of the uses according to the invention correspond to or can be derived from the (preferred) embodiments of the compounds for use according to the invention which are explained above or *vice versa.* (Preferred) embodiments of the methods according to the invention correspond to or can be derived from the (preferred) embodiments of the compounds for use according to the invention which are explained above or *vice versa.* Moreover, the (preferred) embodiments described herein can be arbitrarily combined with each other as long as technically sensible.

The invention will now be described in more detail hereinafter with references to the examples. Further aspects of the present invention are disclosed in the accompanying claims. Unless otherwise stated, all values refer to weight-% (wt.-%).

Examples:

**1. Tested compounds of formula (I)**

**Table 1:** Structures of the compounds of formula (I)

| IUPAC | Structure |
|---|---|
| 3-Hydroxypropyldecanoate | 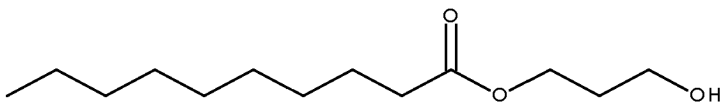 |
| 3-Hydroxypropyldodecanoate | 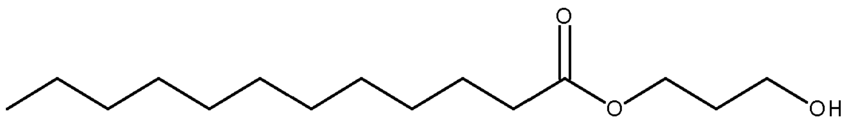 |

The compounds of formula (I) can be obtained, for instance, by following the synthetic approaches described in Synthesis, 2003, (15), 2373-2377; Journal of the Korean Chemical Society, 2002, 46(5); and Tetrahedron Letters, 2015, 56(15), 1973-1975.

The compounds of formula (I) are accessible not only via precursors of petrochemical origin, but advantageously also in a sustainable and/or cost-efficient way from biobased starting materials comprising only non-fossil fuel-based carbon. Assessment of the biobased carbon in a material can be performed through standard test methods. Using radiocarbon and isotope ratio mass spectrometry analysis, the biobased content of materials can be determined. ASTM International, formally known as the American Society for Testing and Materials, has established a standard test method for assessing the biobased content of materials. The ASTM method is designated ASTM D6866. According to a preferred embodiment of the present invention, more than 50% of the carbon atoms contained in a compound of formula (I) are

biobased, based on the total number of carbon atoms contained in said compound of formula (I). More preferably, more than 60%, 70%, 75%, 80%, 85%, or 90% of the carbon atoms contained in a compound of formula (I) are biobased, based on the total number of carbon atoms contained in said compound of formula (I).

## 2. Minimum inhibitory concentration (MIC) and synergistic activity tests

The Minimum Inhibitory Concentration (MIC) test is a test on growth inhibition. The estimation of MICs is executed in 96 well plates. Through the comparison of bacterial growth with positive and negative controls *via* optical density (OD), different concentrations of given test substances are evaluated.

Microorganisms were cultivated under adjusted conditions based on information of the German Collection of Microorganisms and Cell Cultures (DSMZ) and stored in 10 % or 50 % glycerol prior to use. Afterwards, the following method was applied: Growth medium (according to microorganism) was added to each well of the microplate. Then, the test substances, dissolved in DMSO, were added in different concentrations, positive controls (water controls only with medium), solvent controls (DMSO controls only with medium), and negative controls (benchmarks according to organism). Finally, the microorganisms were added in a concentration of 1 to 6 x $10^6$ CFU / ml (CFU = colony forming units). The incubation of the microplates was carried out at appropriate conditions.

According to the resulting growth, substance concentrations are labelled either as inhibiting or not and MIC is defined as the lowest concentration where a complete growth inhibition is visible. Experiments are performed at least twice.

3-Hydroxypropyldodecanoate was active against *Staphylococcus aureus* (32 ppm), *Staphylococcus hominis* (16 ppm), *Staphylococcus epidermidis* (32 ppm), *Corynebacterium xerosis* (32 ppm), *Corynebacterium jeikeium* (2500 ppm), *Streptococcus mutans, Fusobacterium nucleatum, Porphyromonas gingivalis, and Prevotella intermedia* whereas 3-Hydroxypropyldecanoate was active against *Staphylococcus aureus* (125 ppm), *Candida albicans* (125 ppm), *Aspergillus brasiliensis* (500 ppm), *Staphylococcus hominis* (64 ppm), *Staphylococcus epidermidis* (125 ppm), *Corynebacterium xerosis* (125 ppm), *Corynebacterium jeikeium,* (1000 ppm), *Anaerococcus octavius* (1000 ppm), *Streptococcus mutans* (64 ppm), *Fusobacterium nucleatum* (32 ppm), *Porphyromonas gingivalis* (32 ppm), *and Prevotella intermedia (32* ppm), *Malassezia sympodialis* (2500 ppm), *Staphylococcus capitis* (64 ppm), *Cutibacterium acnes* (500 ppm), *Trichophyton mentagrophytes,* and *Brevibacterium epidermidis.* It should be emphasized that *Anaerococcus octavius* represents the category of anaerobic odour causing bacteria. As a matter of fact, it is very hard to find antimicrobial materials which are active against these microorganisms. Those that are usually used for skin applications with low MIC values do not work against *Anaerococcus octavius* at a comparable concentration.

The synergistic activity of the compounds of formula (I) in combination with different further antimicrobial agents was determined by measuring the Minimum Inhibitory Concentrations (MIC) according to the method as described above.

To verify that the tested combinations act synergistically against microorganisms, corresponding experiments were conducted with selected organisms from the list of *Staphylococcus aureus, Candida albicans, Aspergillus brasiliensis, Pseudomonas aeruginosa* and *Escherichia coli.* The synergistic activities of the compounds of formula (I) and different further antimicrobial agents in a mixture of 1:1 (by weight) were determined by measuring the MIC of the mixtures and compare the determined MICs of the mixtures with the MICs of the individual substances, respectively. The calculation of a synergy index gave a quantitative indication of the synergistic effects.

To calculate the synergy index with MIC values, the Kull equation was used as follows:

$$SI = (MIC_{mixture} \times P_A) / MIC_A + (MIC_{mixture} \times P_B) / MIC_B$$

wherein

| | |
|---|---|
| SI | is the Synergy Index according to Kull |
| $MIC_A$ | is the MIC value for Substance A |
| $MIC_B$ | is the MIC value for Substance B |
| $MIC_{mixture}$ | is the MIC value for the mixture of substances A and B |
| $P_A$ | is the proportion of the substance A in the mixture |
| $P_B$ | is the proportion of the substance B in the mixture |

Table 2

| Test substances | MIC in ppm |
|---|---|
| | *Staphylococcus aureus* |
| Benzyl salicylate | 10000 |
| 3-Hydroxypropyldecanoate | 125 |
| Benzyl salicylate 3-Hydroxypropyldecanoate Mixture 50:50 | 125 |
| | Synergy Index |
| | 0.51 |

Table 3

| Test substances | MIC in ppm |
|---|---|
| | *Staphylococcus aureus* |
| 1,2-Hexanediol | 20000 |
| 3-Hydroxypropyldecanoate | 125 |
| 1,2-Hexanediol 3-Hydroxypropyldecanoate Mixture 50:50 | 125 |
| | Synergy Index |
| | 0.50 |

Table 4

| Test substances | MIC in ppm |
|---|---|
| | *Staphylococcus aureus* |
| 1,2-Decanediol | 250 |
| 3-Hydroxypropyldecanoate | 125 |
| 1,2-Decanediol 3-Hydroxypropyldecanoate Mixture 50:50 | 64 |
| | Synergy Index |
| | 0.38 |

Table 5

| Test substances | MIC in ppm |
|---|---|
| | *Staphylococcus aureus* |
| Bisabolol | 125 |
| 3-Hydroxypropyldecanoate | 125 |
| Bisabolol 3-Hydroxypropyldecanoate Mixture 50:50 | 64 |
| | Synergy Index |
| | 0.51 |

Table 6

| Test substances | MIC in ppm |
|---|---|
| | *Staphylococcus aureus* |
| 1,3-Propanediol | 175000 |
| 3-Hydroxypropyldecanoate | 125 |

(continued)

| Test substances | MIC in ppm |
|---|---|
| | ***Staphylococcus aureus*** |
| 1,3-Propanediol 3-Hydroxypropyldecanoate Mixture 50:50 | 125 |
| | **Synergy Index** |
| | 0.50 |

**Table 7**

| Test substances | MIC in ppm |
|---|---|
| | ***Staphylococcus aureus*** |
| Ethylhexylglycerin | 1000 |
| 3-Hydroxypropyldecanoate | 125 |
| Ethylhexylglycerin 3-Hydroxypropyldecanoate Mixture 50:50 | 125 |
| | **Synergy Index** |
| | 0.56 |

**Table 8**

| Test substances | MIC in ppm |
|---|---|
| | ***Staphylococcus aureus*** |
| Chlorphenesin | 5000 |
| 3-Hydroxypropyldecanoate | 125 |
| Chlorphenesin 3-Hydroxypropyldecanoate Mixture 50:50 | 125 |
| | **Synergy Index** |
| | 0.51 |

**Table 9**

| Test substances | MIC in ppm |
|---|---|
| | ***Staphylococcus aureus*** |
| Glyceryl Caprylate | 1000 |
| 3-Hydroxypropyldecanoate | 125 |
| Glyceryl Caprylate 3-Hydroxypropyldecanoate Mixture 50:50 | 125 |
| | **Synergy Index** |
| | 0.56 |

**Table 10**

| Test substances | MIC in ppm |
|---|---|
| | ***Staphylococcus aureus*** |
| Caprylhydroxamic acid | 1000 |
| 3-Hydroxypropyldecanoate | 125 |
| Caprylhydroxamic acid 3-Hydroxypropyldecanoate Mixture 50:50 | 125 |

(continued)

| | **Synergy Index** |
|---|---|
| | 0.56 |

**Table 11**

| **Test substances** | **MIC in ppm** |
|---|---|
| | ***Staphylococcus aureus*** |
| Methylparaben | 2500 |
| 3-Hydroxypropyldecanoate | 125 |
| Methylparaben<br>3-Hydroxypropyldecanoate<br>Mixture 50:50 | 125 |
| | Synergy Index |
| | 0.53 |

**Table 12**

| **Test substances** | **MIC in ppm** |
|---|---|
| | ***Staphylococcus aureus*** |
| Ethylparaben | 1000 |
| 3-Hydroxypropyldecanoate | 125 |
| Ethylparaben<br>3-Hydroxypropyldecanoate<br>Mixture 50:50 | 125 |
| | **Synergy Index** |
| | 0.56 |

**Table 13**

| **Test substances** | **MIC in ppm** |
|---|---|
| | ***Staphylococcus aureus*** |
| Itaconic acid | 5000 |
| 3-Hydroxypropyldecanoate | 125 |
| Itaconic acid<br>3-Hydroxypropyldecanoate<br>Mixture 50:50 | 125 |
| | **Synergy Index** |
| | 0.51 |

**Table 14**

| **Test substances** | **MIC in ppm** |
|---|---|
| | ***Staphylococcus aureus*** |
| 1,2-Nonanediol | 1000 |
| 3-Hydroxypropyldecanoate | 125 |

(continued)

| Test substances | MIC in ppm |
|---|---|
| | ***Staphylococcus aureus*** |
| 1,2-Nonanediol<br>3-Hydroxypropyldecanoate<br>Mixture 50:50 | 125 |
| | **Synergy Index** |
| | 0.56 |

**Table 15**

| Test substances | MIC in ppm |
|---|---|
| | ***Staphylococcus aureus*** |
| 1,2-Heptanediol | 7500 |
| 3-Hydroxypropyldecanoate | 125 |
| 1,2-Heptanediol<br>3-Hydroxypropyldecanoate<br>Mixture 50:50 | 125 |
| | **Synergy Index** |
| | 0.51 |

**Table 16**

| Test substances | MIC in ppm |
|---|---|
| | ***Staphylococcus aureus*** |
| 1,2-Pentanediol | 35000 |
| 3-Hydroxypropyldecanoate | 125 |
| 1,2-Pentanediol<br>3-Hydroxypropyldecanoate<br>Mixture 50:50 | 125 |
| | **Synergy Index** |
| | 0.50 |

**Table 17:** *The MIC value of hydroxyethoxyphenyl butanone was determined as being larger than 10000 ppm. For this calculation, a MIC value of 10000 ppm was used. The respective SI value is calculated as 0.51. The actual SI value will be lower.

| Test substances | MIC in ppm |
|---|---|
| | ***Staphylococcus aureus*** |
| Hydroxyethoxyphenyl butanone | > 10000* |
| 3-Hydroxypropyldecanoate | 125 |
| Hydroxyethoxyphenyl butanone<br>3-Hydroxypropyldecanoate<br>Mixture 50:50 | 125 |
| | **Synergy Index** |
| | 0.51* |

Table 18

| Test substances | MIC in ppm |
|---|---|
| | ***Staphylococcus aureus*** |
| Methylheptylglycerin | 2500 |
| 3-Hydroxypropyldecanoate | 125 |
| Methylheptylglycerin<br>3-Hydroxypropyldecanoate<br>Mixture 50:50 | 125 |
| | **Synergy Index** |
| | 0.53 |

Table 19

| Test substances | MIC in ppm |
|---|---|
| | ***Staphylococcus aureus*** |
| Raspberry Ketone | 5000 |
| 3-Hydroxypropyldecanoate | 125 |
| Raspberry Ketone<br>3-Hydroxypropyldecanoate<br>Mixture 50:50 | 125 |
| | **Synergy Index** |
| | 0.51 |

Table 20

| Test substances | MIC in ppm |
|---|---|
| | ***Staphylococcus aureus*** |
| 1,2-Octanediol | 5000 |
| 3-Hydroxypropyldecanoate | 125 |
| 1,2-Octanediol<br>3-Hydroxypropyldecanoate<br>Mixture 50:50 | 125 |
| | **Synergy Index** |
| | 0.51 |

### 3. Reduction of odour formation

The formation of sweat odour was investigated in an axillary microbiome *ex vivo* model that is based on human axillary sweat and results were confirmed by a single blind placebo-controlled in vivo study with a total of 60 human volunteers. Fresh sweat was incubated in the model and shows a characteristic development over time (0h, 24h, 48h). This includes accumulation of compounds related to sweat odour (measured with GC-MS) going along with the development of the typical sweat odour (assessed by a sensory panel), and a microbiological analysis.

### 3.1 Microbial load

The resulting sweat odour is the result of microbial activity. Those microorganisms that can metabolize the nutrients contained in the sweat can also increase their cell number. Untreated sweat starts with a cell number of about $10^6$ cells/ml. These numbers increase by 1.5 to 2 numbers of magnitude during incubation, both for aerobic as well as anaerobic cell count. The following **Table 21** shows aerobic and anaerobic cell counts untreated and in the presence of 1%

and 0.5% of 3-hydroxypropyldecanoate.

**Table 21:** Aerobic and anaerobic cell counts

| Timepoint | Conditions | Aerobic | Anaerobic |
|---|---|---|---|
| immediately | untreated | 2.30E+06 | 1.50E+06 |
| 24h | untreated | 2.80E+08 | 7.60E+07 |
| | 3-Hydroxypropyldecanoate 1% | 3.20E+07 | 3.10E+07 |
| | 3-Hydroxypropyldecanoate 0.5% | 1.80E+07 | 1.60E+07 |
| 48h | untreated | 7.80E+07 | 3.70E+07 |
| | 3-Hydroxypropyldecanoate 1% | 6.30E+06 | 3.20E+06 |
| | 3-Hydroxypropyldecanoate 0.5% | 5.20E+06 | 4.20E+06 |

### 3.2 16S rRNA gene sequencing

16S rRNA gene sequencing revealed an inhibiting effect on *Anaerococcus* (24h: untreated 17.6%, 3-hydroxypropyldecanoate 1%: 15.7%, 3-hydroxypropyldecanoate 0.5%: 13.8%; 48h: untreated 33.0%, 3-hydroxypropyldecanoate 1%: 16.7%, 3-hydroxypropyldecanoate 0.5%: 16.4%) and unassigned genera (24h: untreated 23.4%, 3-hydroxypropyldecanoate 1%: 0.4%, 3-hydroxypropyldecanoate 0.5%: 0.3%; 48h: untreated 25.4%, 3-hydroxypropyldecanoate 1%: 11.4%, 3-hydroxypropyldecanoate 0.5%: 0.1%).

### 3.3 Odour evaluation

The addition of 3-hydroxypropyldecanoate at 1% and 0.5% to the fresh sweat and incubation at exactly the same conditions as untreated sweat shows the functional impact of this ingredient. The sensory analysis revealed a clear reduction of odour development in comparison to untreated when 3-hydroxypropyldecanoate is present. This sensory impression was confirmed analytically with a drastically reduced formation of odorous products in the form of volatile organic acids (cf. section 3.4 below). **Table 22** below shows the result of a sensory panel consisting of 7 experienced panelists.

**Table 22:** Odour rating as an evaluation of smell intensity by a sensory panel, odour values: 0 (no sweat odour) to 10 (very strong odour)

| | immediately | 48h | | |
|---|---|---|---|---|
| | | untreated | 3-Hydroxypropyldecanoate 0.5% | 3-Hydroxypropyldecanoate 1% |
| Mean | 2.0 | 5.9 | 1.6 | 1.3 |
| Standard deviation | 1.4 | 1.4 | 1.8 | 0.7 |

### 3.4 Odorous compounds

Samples have been purified by trichloromethane extraction. Volatile compounds were quantified using GC-MS (TIC mode). The results are shown in **Table 23** below. Through their volatile character, these compounds are described to shape the individual character of sweat, hereby strongly influencing the personal reception of malodour.

**Table 23**

| [amounts in ppm] | 0h | 24h | | | 48h | | |
|---|---|---|---|---|---|---|---|
| **Compound** | untreated | untreated | 3-Hydroxypropyl-decanoate 1% | 3-Hydroxypropyl-decanoate 0.5% | untreated | 3-Hydroxypropyl-decanoate 1% | 3-Hydroxypropyl-decanoate 0.5% |
| Acetic Acid | 0.59 | 10.01 | 2.44 | 3.22 | 22.34 | 3.54 | 4.50 |
| Propanoic Acid | 0.28 | 3.92 | 0.61 | 0.61 | 4.85 | 0.61 | 0.64 |
| Isobutyric Acid | 0.02 | 0.55 | 0.03 | 0.02 | 2.80 | 0.03 | 0.03 |
| Butyric Acid | 0.53 | 15.12 | 2.65 | 2.42 | 44.79 | 2.11 | 1.97 |
| Methylbutyric Acid, 2- | 0.12 | 1.50 | 0.13 | 0.12 | 5.83 | 0.14 | 0.13 |
| Valeric Acid | 0.24 | 0.47 | 0.34 | 0.38 | 1.61 | 0.33 | 0.33 |
| Capronic Acid | 0.34 | 0.68 | 0.64 | 0.86 | 7.97 | 0.57 | 0.79 |
| Hexenoic Acid, 3-Methyl-2- | 0.02 | 0.43 | 0.09 | 0.07 | 0.31 | 0.11 | 0.12 |
| Indole | 0.03 | 4.95 | 0.05 | 0.05 | 4.35 | 0.05 | 0.04 |
| **Sum** | **2.18** | **37.62** | **6.99** | **7.74** | **94.85** | **7.49** | **8.55** |

**4. Formulation examples**

Cosmetic formulations (compositions) - amounts are indicated as % by weight for all formulations.

DEODORANTS

**Table 24:** Deodorant Spray

| Ingredients | EU INCI | Amount |
|---|---|---|
| Aqua/Water | Aqua | Ad 100 |
| Volarest™ FL | Acrylates/Beheneth-25 Methacrylate copolymer | 1.2 |
| Dracorin® GOC | Glyceryl oleate citrate; Caprylic/Capric triglyceride | 2.0 |
| SymDeo® Plus | Lauryl alcohol, Phenoxyethanol; 2-Benzylheptanol; Decylene Glycol | 0.5 |
| 3-Hydroxypropyldecanoate | | 0.8 |
| SymOcide® PS | Phenoxyethanol; Decylene Glycol; 1,2-Hexanediol | 0.8 |
| Dow Corning® 245 Fluid | Cyclomethicone | 5.0 |
| Isopropyl Myristate | Isopropyl myristate | 2.0 |
| Farnesol | Farnesol | 0.25 |
| Ethylhexylglycerin | Ethylhexylglycerin | 0.2 |
| SymDeo® B125 | 2-Methyl 5-Cycloheylpentanol | 0.15 |
| Tego® Cosmo P 813MB | Polyglyceryl-3 Caprylate | 0.25 |
| Fragrance composition | Parfum | 0.8 |
| Frescolat® ML nat | Menthyl Lactate | 0.2 |
| Sodium Hydroxide, 10% solution | Aqua; Sodium hydroxide | 0.55 |

**Table 25:** Cool Relief Deo Plus

| Ingredients | INCI | Amount |
|---|---|---|
| Aqua / Water | Aqua | Ad 100 |
| Veegum® HS Granules | Magnesium Aluminium Silicate | 1.0 |
| Glycerin | Glycerin | 5.0 |
| EDTA NA2 | Disodium EDTA | 0.1 |
| Dracorin® 100 SEP | Glyceryl Stearate, PEG-100 Stearate | 6.0 |
| Lanette 16 | Cetyl alcohol | 2.0 |
| Eumulgin® B2 | Ceteareth-20 | 6.0 |
| Eutanol® G | Octyldodecanol | 8.0 |
| Dow Cornng FZ-3196 Volatile | Caprylyl Methicone | 4.0 |
| Tween 20 | Polysorbate20 | 0.3 |
| SymDeo® B125 | 2-Methyl 5-Cyclohexylpentanol | 0.3 |
| 3-Hydroxypropyldecanoate | | 0.2 |
| Dragosantol® 100 | Bisabolol | 0.05 |
| Aqua/Water | Aqua | 15.0 |
| Locron® P | Aluminium Chlorohydrate | 15.0 |
| SymOcide® PS | Phenoxyethanol, Decylene Glycol, 1,2-Hexanediol | 1.0 |

(continued)

| Ingredients | INCI | Amount |
|---|---|---|
| Frescolat® ML | Menthyl Lactate | 0.5 |
| Vitacel® CS020 | Cellulose | 1.0 |
| Extrapone® Rose Quartz GW | Aqua, Glycerin, Xanthan Gum, Quartz Powder | 1.0 |
| Fragrance composition | Parfum | 1.0 |
| Sodium hydroxide 10% solution | Aqua, Sodium hydroxide | 0.5 |

**Table 26:** Deodorant stick

| Ingredients | INCI | Amount |
|---|---|---|
| Sodium stearate | Sodium stearate | 8.0 |
| PPG-3 Myristyl ether | PPG-3 Myristyl ether | 70.0 |
| 1,2-propylene glycol | 1,2-propylene glycol | 10.0 |
| 1,1-dimethyl-3-phenylpropanol | 1,1-dimethyl-3-phenylpropanol | 0.2 |
| 2-butyloctanoic acid | 2-butyloctanoic acid | 0.2 |
| MAA-2 Salt | | 0.2 |
| MAA-9 Acid | | 0.4 |
| Fragrance composition | Parfume | 0.6 |
| Water | Aqua | ad 100 |
| SymDeo® Plus | Lauryl Alcohol, Phenoxyethanol, 2-Benzylheptanol, Decylene Glycol | 0.5 |
| 3-Hydroxypropyldodecanoate | | 0.4 |
| N-Ethyl-p-menthane-3-carboxamide (Cooling Agent WS-3) | Ethyl Menthane Carboxamide | 0.3 |

**Table 27:** Antiperspirant/deodorant roll-on

| Ingredients | INCI | Amount |
|---|---|---|
| Dragosantol® 100 | Bisabolol | 0.1 |
| Ethanol 96 % | Ethanol | 30.0 |
| Farnesol | Farnesol | 0.5 |
| Fragrance composition | Parfum | 1.5 |
| Frescolat®ML cryst. | Menthyl Lactate | 0.2 |
| Irgasan® DP 300 | Triclosan | 0.3 |
| Natrosol™ 250 HHR | Hydroxyethyl-cellulose | 0.3 |
| Solubilizer | PEG-40 Hydrogenated Castor Oil, Trideceth-9, Water (Aqua) | 2.0 |
| 3-Hydroxypropyldecanoate | | 0.2 |
| SymDeo® B125 | 2-Methyl 5-Cyclohexylpentanol | 0.5 |
| Water (demineralized) | Water (Aqua) | ad 100 |
| Zirkonal L 450 | Aluminium Zirconium Pentachloro-hydrate (40 % aqueous solution) | 37.0 |

**Table 28:** Deodorant formulation in the form of a roll-on gel

| Ingredients | INCI | Amount |
|---|---|---|
| 1,3-butylene glycol | 1,3-butylene glycol | 2.0 |
| Cremophor ® CO 40 Surfactant | PEG-40-hydrogenated castor oil | 2.0 |
| Hydroxyethylcellulose | Hydroxyethylcellulose | 0.5 |
| Fragrance composition | Parfum | 0.3 |
| 1,3-propanediol | 1,3-propanediol | 0.5 |
| SymGuard® CD | 3-Phenylpropanol, o-cymen-3-ol, Decylene glycol | 0.4 |
| 3-Hydroxypropyldecanoate | | 0.2 |
| Ethylhexyl glycerin | Ethylhexyl glycerin | 0.1 |
| Water | | ad 100 |

**Table 29:** Clear deo anti-perspirant roll-on

| Ingredients | INCI | Amount |
|---|---|---|
| Methocel™ E4M Premium | Hydroxypropyl Methylcellulose | 0.5 |
| Water | Water (Aqua) | ad 100 |
| Neo-PCL Water Soluble N | Trideceth-9, PEG-5 Ethylhexanoate, Water (Aqua) | 1.0 |
| Solubilizer | PEG-40 Hydrogenated Castor Oil, Trideceth-9, Propylene Glycol, Water (Aqua) | 3.0 |
| Deolite | Dimethyl Phenylpropanol, Pentylene Glycol | 0.5 |
| Locron® LW | Aluminium Chlorohydrate | 25.0 |
| Aloe Vera Gel Concentrate 10/1 | Aloe Barbadensis Leaf Juice | 1.0 |
| 1,2-Propylene Glycol 99 P GC | Propylene Glycol | 4.0 |
| 3-Hydroxypropyldecanoate | | 0.9 |
| Ethanol 96 % | Alcohol Denat. | 30.0 |
| Fragrance composition | Parfum | 1.0 |

GELS

**Table 30:** After Shave Tonic

| Ingredients | INCI | Amount |
|---|---|---|
| SymSol® PF-3 | Water (Aqua), Pentylene Glycol, Sodium Lauryl Sulfoacetate, Sodium Oleoyl Sarcosinate, Sodium Chloride, Disodium Sulfoacetate, SodiumOleate, Sodium Sulfate | 3.0 |
| SymSitive® 1609 | Pentylene Glycol, 4-t-Butylcyclohexanol | 1.0 |
| Frescolat® ML | Menthyl Lactate | 0.3 |
| Glycerol 99,5 P. | Glycerol | 5.0 |
| Water | Water (Aqua) | ad 100 |
| Extrapone® Glacier Water GW | Glycerol, Water (Aqua) | 1.0 |
| SymCalmin® | Butylene Glycol, Pentylene Glycol, Hydroxyphenyl Propamidobenzoic Acid | 0.5 |
| Dragosine® | Carnosine | 0.1 |

(continued)

| Ingredients | INCI | Amount |
|---|---|---|
| Hydrolite® 5 green | Pentylene Glycol | 5.0 |
| Ethanol 96 % | Alcohol Denat. | 5.0 |
| Colour Pigment | Colour Pigment | 0.05 |
| Fragrance composition | Parfum | 0.15 |
| 3-Hydroxypropyldodecanoate | | 0.05 |
| Alpha Tocopherol DL | Tocopherol | 0.2 |

**Table 31:** Flash SOS Aloe Gelée

| Ingredients | EU INCI | Amount |
|---|---|---|
| Dracorin® GOC | Glyeryl Oleate Citrate, Caprylic/Capric Triglyceride | 0.6 |
| SymMollient® PDCC | Propanediol Dicaprylate/Caprate | 6.0 |
| Dragoxat® 89 | Ethylhexyl Isononanoate | 5.0 |
| PCL-Liquid® 100 | Cetearyl Ethylhexanoate | 1.5 |
| Frescolat® X-Cool | Menthyl Ethylamido Oxalate | 1.0 |
| SymRelief® 100 | Bisabolol, Zingiber Officinale Root Extract | 0.1 |
| SymDecanox™HA | Caprylic/Capric Triglyceride, Hydroxymethoxyphenyl Decanone | 0.8 |
| Alpha Tocopherol DL | Tocopherol | 0.1 |
| Carbopol® Ultrez 20 Polymer | Acrylates/C10-30 Alkyl Acrylate Crosspolymer | 0.4 |
| Aqua/Water | Aqua | Ad 100 |
| Glycerin | Glycerin | 3.0 |
| SymMollient® W/S | Trideceth-9, PEG-5 Isononanoate, Aqua | 1.0 |
| SymDiol® 68 | 1,2-Hexanediol, Caprylyl Glycol | 0.5 |
| SymSave® H | Hydroxyacetophenone | 0.5 |
| Hydroviton® PLUS 2290 | Aqua, Pentylene Glycol, Glycerin, Fructose, Urea, Citric acid, Sodium hydroxide, Maltose, Sodium PCA, Sodium Chloride, Sodium Lactate, Trehalose, Allantoin, Sodium hyaluronate, Glucose | 2.5 |
| 3-Hydroxypropyldecanoate | | 1.0 |
| Aloe Vera Gel Concentrate | Aloe Barbadensis Leaf Juice | 1.5 |
| Sodium Hydroxide 10% solution | Aqua, Sodium hydroxide | 0.7 |
| Fragrance composition | Parfum | 0.5 |
| Color | | 0.03 |

**Table 32:** After Shave

| Ingredients | EU INCI | Amount |
|---|---|---|
| Dracorin® GOC | Glycery Oleate Citrate, Caprylic/Capric Triglyceride | 1.0 |
| Dragoxat® 89 | Ethylhexyl Isononanoate | 5.0 |
| SymMollient® S | Cetearyl Nonanoate | 1.0 |
| Tocopheryl Acetate | Tocopheryl Acette | 0.25 |
| Aqua/Water | Aqua | Ad 100 |
| Pemulen™ TR-2 | Acrylates/C10-30 Alkyl Acrylate Crosspolymer | 0.3 |

(continued)

| Ingredients | EU INCI | Amount |
|---|---|---|
| Aloe Vera Gel concentrate 10/1 | Aloe Barbadensis leaf juice | 0.5 |
| SymCare® W2 | PEG-40 Hydrogenated castor oil, Trideceth-9, Pentylene Glycol, 4-t-Butylcyclohexanol, Propylene Glycol, Aqua, Hydroxyphenyl propamidobenzoic acid | 1.0 |
| Glycerin | Glycerin | 3.0 |
| SymDiol® 68 | 1,2-Hexanediol, Caprylyl Glycol | 0.5 |
| SymSave® H | Hydroxyacetophenone | 0.5 |
| SymGlucan® | Aqua, Glycerin, 1,2-Hexanediol, Caprylyl Glycol, Beta-Glucan | 0.2 |
| Extrapone® Glacier Water GW | Glycerin, Aqua | 1.0 |
| Allantoin | Allantoin | 0.15 |
| 3-Hydroxypropyldodecanoate | | 0.25 |
| Sodium Hydroxide 10% solution | Aqua, Sodium Hydroxide | 0.54 |
| Fragrance composition | Parfum | 1.0 |
| Frescolat® ML cryst | Menthyl Lactate | 0.5 |

**Table 33:** Frosty Facial Foam

| Ingredients | EU INCI | Amount |
|---|---|---|
| Aqua Water | Aqua | Ad 100 |
| Edeta B Powder | Tetrasodium EDTA | 0.1 |
| Glycerin | Glycerin | 3.0 |
| Potassium Hydroxide, 85% Pellets | Potassium hydroxide | 3.5 |
| Myristic Acid 99% | Myristic acid | 6.0 |
| Lauric Acid, 99% | Lauric acid | 6.0 |
| Stearic Acid, 98% | Stearic acid | 11.0 |
| Miranol® C2M CONC NP | Disodium Cocoamphodiacetate | 4.0 |
| SymMollient® W/S | Trideceth-9, PEG-5 Isononanoate, Aqua | 2.0 |
| Fragrance composition | Parfum | 0.3 |
| Frescolat® MGA Plus | Menthone Glycerin acetal, Menthol | 0.5 |
| SymOcide® OS | Phenoxyethanol, Decylene Glycol, 1,2-Hexanediol | 0.8 |
| 3-Hydroxypropyldecanoate | | 0.6 |
| Colour, 0.1% aq. | CI42090 | 0.5 |

**Table 34:** Hydration Face Gel, water clear

| Ingredients | EU INCI | Amount |
|---|---|---|
| Aqua/Water | Aqua | Ad 100 |
| Gamma-Max | Sodium Polyglutamate | 0.1 |
| Glycerin 99.5% | Glycerin | 3.0 |
| Genu® Gum | Gellan Gum | 1.0 |
| SymOcide® OS | Phenoxyethanol, Decylene Glycol, 1,2 Hexanediol | 0.6 |
| SymHair ® Force 1631 | Pentylene Glycol, Isochrysis Galbana Extract | 2.5 |

(continued)

| Ingredients | EU INCI | Amount |
|---|---|---|
| 3-Hydroxypropyldodecanoate | | 0.6 |
| Raspberry Ketone | | 0.1 |
| SymHair ® Shape&Color | Cetearyl Nonanoate, Triticum Vulgare (Wheat) Germ Oil, Caprylic/-Capric Triglyceride, Linoleic Acid, Triticum Vulgare (Wheat) Bran Extract, Triticum Vulgare (Wheat) Germ Extract, Camellia Oleifera Seed Oil | 0.5 |

**Table 35:** Intimate Sensi-Gel

| Ingredients | EU INCI | Amount |
|---|---|---|
| Aqua/Water | Aqua | Ad 100 |
| Sodium Benzoate | Sodium benzoate | 0.3 |
| Keltrol® CG-T | Xanthan Gum | 0.2 |
| Cyamopsis Tetragonolobus Gum | Cyamopsis Tetragonolobus Gum | 0.1 |
| Glycerin | Glycerin | 4.0 |
| Tego® Betain F50 | Cocamidopropyl betaine | 14.0 |
| Solubilizer | PEG-40 Hydrogenated castor oil, Trideceth-9, Propylene Glycol, Aqua | 3.0 |
| Hydrolite® 6 | 1,2 Hexanediol | 1.0 |
| SymGuard® CD | Phenylpropanol, o-cymen-5-ol, Decylene Glycol | 0.3 |
| SymRelief® 100 | Bisabolol, Zingiber officinale root extract | 0.1 |
| Fragrance composition | Parfum | 0.2 |
| 3-Hydroxypropyldodecanoate | | 0.7 |
| Frescolat® X-cool | Menthyl ethylamido oxalate | 0.4 |
| Plantacare® 2000 UP | Decyl Glucoside | 2.0 |
| Lactic acid 90% Nat. | Lactic Acid, Aqua | 0.3 |

**Table 36:** Mild feminine wash

| Ingredients | EU INCI | Amount |
|---|---|---|
| Aqua/Water | Aqua | Ad 100 |
| SymSave® H | Hydroxyacetophenone | 0.5 |
| Keltrol® CG-T | Xanthan Gum | 0.5 |
| Glycerin | Glycerin | 2.0 |
| Methylheptylglycerin | | 0.25 |
| Propylene Glycol | Propylene Glycol | 3.0 |
| 3-Hydroxypropyldecanoate | | 1.0 |
| Tego® Betain F50 | Cocamidopropyl betaine | 14.0 |
| SymGuard® CD | Phenylpropanol, o-cymen-5-ol, Decylene Glycol | 0.3 |
| Plantacare 2000 UP | Decyl Glucoside | 2.0 |
| Extrapone® Witch Hazel GW | Glycerin, Aqua, Hamamelis Virginiana Bark/- Leaf/Twig Extract | 1.0 |
| Solubilizer | PEG-40 Hydrogenated castor oil, Trideceth-9, Propylene Glycol, Aqua | 1.5 |
| Fragrance composition | Parfum | 0.2 |

(continued)

| Ingredients | EU INCI | Amount |
|---|---|---|
| SymCalmin® | Pentylene Glycol, Butylene Blycol, Hydroxyphenyl propamidobenzoic acid | 1.0 |
| Lactic acid 90% Nat. | Lactic Acid, Aqua | 0.32 |

**Table 37:** Sprayable Anti Cellulite Gel

| Ingredients | INCI | Amount |
|---|---|---|
| Aqua/Water | Aqua | Ad 100 |
| Stabileze QM | PVM / Ma Decadiene Crosspolymer | 0.25 |
| Sodium Hydroxide 10% aqueous solution | Aqua Sodium hydroxide | 0.4 |
| Coffein pure | Caffeine | 0.5 |
| Extrapone® Horse Chestnut | Propylene Glycol, Water (Aqua), Aesculus Hippocastanum (Horse Chestnut) Seed Extract, Glucose, Lactic Acid | 1.0 |
| Hydrolite® 5 | Pentylene Glycol | 3.0 |
| 1,3 Butylene Glycol | Butylene Glycol | 5.0 |
| Biotive® Esculin Sesquihydrate | Esculin | 0.3 |
| Preservative | Phenoxyethanol, Methylparaben, Ethylparaben, Butylparaben, Propylparaben, Isobutylparaben | 0.8 |
| Ethanol 96 % | Alcohol Denat, | 10.0 |
| Solubilizer | PEG-40 Hydrogenated Castor Oil, Trideceth-9, Water (Aqua) | 0.5 |
| Fragrance composition | Parfum | 0.2 |
| 3-Hydroxypropyldecanoate | | 0.2 |
| Benzyl Salicylate | Benzyl Salicylate | 0.1 |
| Chlorphenesin | Chlorphenesin | 0.1 |

HAIR CARE

Shampoo

**Table 38:** Anti Oil Shampoo

| Ingredients | INCI | Amount |
|---|---|---|
| Aqua/Water | Aqua | Ad 100 |
| SymSave® H | Hydroxyacetophenone | 0.5 |
| Ucare ™ Polymer JR-400 | Polyquaternium-10 | 0.25 |
| Dehyton® K | Cocamidopropyl Betaine | 10.0 |
| SymDiol® 68 | 1,2-Hexanediol, Caprylyl Glycol | 0.5 |
| SymClariol | Decylene Glycol | 0.3 |
| Texapon HBN | Sodium Laureth Sulfate | 30.0 |
| Amille GCK-12H | Potassium cocoyl Glycinate (and) Potassium cocoate (and) Aqua | 6.0 |
| Rewopal® PEG 6000DS | PEG-150 Distearate | 3.0 |

(continued)

| Ingredients | INCI | Amount |
|---|---|---|
| Frescolat® ML | Menthyl Lactate | 0.2 |
| SymControl® Scalp | Water (Aqua), Glycerin, Mannitol, Tetraselmis Suecica Extract | 2.0 |
| SymMollient® W/S | Trideceth-9, PEG-5-Isononanoate, Aqua | 2.0 |
| Cremophor® CO 40 Surfactant | PEG-40 Hydrogenated Castor oil | 1.0 |
| Cosmetic Color Blue 1% sol. | CI 42090 | 0.33 |
| Food Color Tartrazine 0.5% sol | CI 19140 | 0.12 |
| Fragrance composition | Parfum | 1.0 |
| 3-Hydroxypropyldecanoate | | 0.4 |
| Extrapone® Green Tea GW | Glycerin, Aqua, Camellia sinensis Leaf Extract | 0.1 |
| Citric Acid 30% sol. | Aqua, Citric acid | 0.15 |

**Table 39:** Refreshing Green Body Jello

| Ingredients | INCI | Amount |
|---|---|---|
| Aqua/Water | Aqua | Ad 100 |
| Glycerin 99.5% | Glycerin | 58.7 |
| Genugel® Carrageenan CG-130 | Carrageenan | 1.0 |
| Texapon® NSO UP | Sodium Laureth Sulfate | 15.0 |
| SymOcide® PS | Phenoxyethanol, Decylene Glycol, 1,2-Hexanediol | 0.8 |
| Frescolat® MGA Plus | Menthone Glycerin Acetal, Menthol | 1.0 |
| 3-Hydroxypropyldodecanoate | | 1.0 |
| Actipone Grape Seed GW | Aqua, Glycerin, Vitis Vinifera Seed Extract | 0.5 |
| SymHair Restore | Glyerin, Triticum vulgare protein, Aqua | 1.0 |
| Color | | 1.0 |
| Fragrance composition | Parfum | 1.0 |

**Table 40:** Antidandruff Shampoo

| Ingredients | INCI | Amount |
|---|---|---|
| Water, distilled | Water (Aqua) | ad 100 |
| Polymer JR 400 | Polyquaternium-10 | 0.4 |
| Texapon® NSO IS | Sodium Laureth Sulfate | 40.0 |
| Dehyton K | Cocamidopropyl Betaine | 8.0 |
| Plantacare 2000 UP | Decyl Glucoside | 2.0 |
| Citric Acid 10 % solution | Citric Acid | 0.8 |
| Frescolat® X-cool | Menthyl Ethylamido Oxalate | 0.5 |
| SymTriol® | Caprylyl Glycol, 1,2-Hexanediol, Methylbenzyl Alcohol | 0.8 |
| 3-Hydroxypropyldecanoate | | 0.3 |
| Crinipan® AD | Climbazole | 0.5 |
| SymMollient® W/S | Trideceth-9, PEG-5 Isononanoate, Water (Aqua) | 2.0 |
| Tinogard® Q | Tri(tétramethylhydroxypiperodonol) Citrate | 0.02 |
| Sodium Chloride | Sodium Chloride | 0.5 |

(continued)

| Ingredients | INCI | Amount |
|---|---|---|
| Colour I | Colour | 0.3 |
| Colour II | Colour | 0.1 |
| Perfume oil PO1; PO2; PO3; PO4 or PO5 | Fragrance | 0.4 |

**Table 41:** Wild Berries Shower Polish

| Ingredients | EU INCI | Amount |
|---|---|---|
| Aqua | Aqua | Ad 100 |
| Glycerin 99.5% | Glycerin | 3.0 |
| Genuvisco® CG-131 | Carrageenan | 0.7 |
| Keltrol® CG-SFT | Xanthan Gum | 0.3 |
| 3-Hydroxypropyldecanoate | | 1.5 |
| Amisoft CS-22 | Sodium Cocoyl Glutamate, Disodium Cocoyl Glutamate | 20.0 |
| Eucarol AGE/EC | Disodium Coco-Glucoside Citrate | 7.0 |
| SymOcide PS | Phenoxyethanol, Decylene Glycol, 1,2 Hexanediol | 1.0 |
| Jojoba oil | Simmondsia Chinensis (Jojoba) Seed Oil | 8.0 |
| SymControl® Scalp | Water (Aqua), Glycerin, Mannitol, Tetraselmis Suecica Extract | 2.0 |
| Actipone® Goji Berry GW | Water (Aqua), Glycerin, Lycium Barbarum Fruit Extract | 2.0 |
| Vitacel® CS250g | Cellulose | 2.0 |

**Table 42:** Fresh Hair shampoo

| Ingredients | EU INCI | Amount |
|---|---|---|
| Aqua | Aqua | Ad 100 |
| EDTA B Powder | Disodium EDTA | 0.1 |
| Nativacare™ 5600 | Zea Mays (Corn Starch) | 2.0 |
| Rheocare® XGN | Xanthan Gum | 0.5 |
| Texapon® NSO UP | Sodium Laureth sulfate | 35.0 |
| Dehyton® K | Cocamidopropyl betaine | 8.0 |
| 3-Hydroxypropyldecanoate | | 0.6 |
| Methylheptylglycerin | | 0.15 |
| Plantacare® 2000 UP | Decyl Glucoside | 4.0 |
| Fragrance composition | Parfum | 1.0 |
| SymSave® H | Hydroxyacetophenone | 0.5 |
| Hydrolite® 6 | 1,2-Hexanediol | 1.0 |
| SymClariol® | Decylene Glycol | 0.3 |
| Frescolat® ML cryst | Menthyl Lactate | 1.0 |
| SymCalmin® | Pentylene Glycol, Butylene Glycol, Hydroxyphenyl propamidobenzoic acid | 0.2 |
| Crinipan® AD | Climbazole | 0.2 |
| Propylene Glycol | Propylene Glycol | 2.0 |

(continued)

| Ingredients | EU INCI | Amount |
|---|---|---|
| SymHair® Shield | Pentylene Glycol, Aqua, Glycerin, Triticum vulgare bran extract, 1,2-Hexanediol, Capryly glycol | 1.0 |
| Sodium Hydroxide 10% solution | Aqua, Sodium hydroxide | 2.2 |
| Actipone® Alpha-Pulp | Aqua, Butylene Glycol, Malic acid, Actinidia chinensis fruit extract, citrus aurantium dulcis juice, citrus paradise juice,pyrus malus juice | 0.2 |

EMULSIONS

**Table 43:** Skin soothing lotion

| Ingredients | INCI | Amount |
|---|---|---|
| Abil 350 | Dimethicone | 2.0 |
| Allantoin | Allantoin | 0.2 |
| Carbopol Ultrez-10 | Carbomer | 0.1 |
| Ceramide BIO* | Cetylhydroxyproline Palmitamide | 0.1 |
| Citric Acid 10% sol, | Citric Acid | 0.4 |
| Emulsiphos® | Potassium Cetyl Phosphate, Hydrogenated Palm Glycerides | 2.0 |
| Extrapone® Green Tea GW | Glycerin, Water (Aqua), Camellia Sinensis Leaf Extract | 0.2 |
| Extrapone® Rosemary GW | Glycerin, Water (Aqua), Rosmarinus officinalis (Rosemary) Leaf Extract | 0.3 |
| Fragrance composition | Parfum | 0.3 |
| Glycerol 85 % | Glycerin | 2.0 |
| Glyceryl Stearate | Glyceryl Stearate | 2.0 |
| Isodragol® | Triisononanoin | 2.0 |
| 3-Hydroxypropyldodecanoate | | 0.2 |
| Keltrol RD | Xanthan Gum | 0.1 |
| Lanette O | Cetearyl Alcohol | 3.0 |
| Neo PCL wssl, N | Trideceth-9, PEG-5 Ethylhexanoate, Water | 1.0 |
| PCL Liquid 100 | Cetearyl Ethylhexanoate | 5.0 |
| PCL Solid | Stearyl Heptanoate, Stearyl Caprylate | 2.0 |
| Propylene Glycol | Propylene Glycol | 5.0 |
| Sodium Hydroxide 10% aqueous solution | Sodium Hydroxide | 0.3 |
| SymCalmin® | Pentylene Glycol, Butylene Glycol, Hydroxyphenyl Propamidobenzoic Acid | 2.0 |
| SymMatrix® | Maltodextrin, Rubus Fruticosus (Blackberry) Leaf Extract | 0.1 |
| SymOcide® C | Hydroxyacetophenoneo-cymen-5-ol | 0.1 |
| 2-Phenoxyethyl Alcohol Anisic Acid | Phenoxyethanol, Anisic Acid | 0.4 |
| SymSitive®1609 | Pentylene Glycol, 4-t-Butylcyclohexanol | 1.5 |
| Water (demineralized) | Water (Aqua) | ad 100 |

**Table 44:** Eye Cream for Mature Skin

| Ingredients | EU INCI | Amount |
|---|---|---|
| Water | Aqua | Ad 100 |
| Dermosoft® 1288 | Glycerin, Water, Sodium Levulinate, Sodium Anisate | 3.5 |
| Glycerin | Glycerin | 3.0 |
| Sisterna SP30-C | Sucrose Distearate | 3.0 |
| Lanette® O | Cetearyl Alcohol | 2.0 |
| Sisterna SP70-C | Sucrose Stearate | 1.0 |
| Keltrol® CG-SFT | Xanthan Gum | 0.3 |
| 3-Hydroxypropyldecanoate | | 0.4 |
| Amisoft® HS-11P(F) | Sodium Stearoyl Glutamate | 0.2 |
| Genuvisco® CG-131 | Carrageenan | 0.1 |
| Cacao Butter | Cacao Butter | 2.0 |
| Avocado Oil | Persea Gratissima (Avocado) Oil | 2.0 |
| Plantoil Triglyceride | Caprylic/Capric Triglyceride | 17.0 |
| Fragrance composition | Parfum | 1.0 |
| SymOcide® PH | Phenoxyethanol, Hydroxyacetophenone, Caprylyl Glycol, Water(Aqua) | 1.0 |
| Citric Acid, 10% sol | Aqua, Citric acid | 3.5 |

**Table 45:** Refreshing and Soothing After Sun Cream

| Ingredients | EU INCI | Amount |
|---|---|---|
| Dracorin® CE | Glyceryl Stearate Citrate | 2.5 |
| Cutina® GMS V | Glyceryl Stearate | 2.5 |
| Coconut Oil | Cocos Nucifera Oil | 3.0 |
| SymMollient® PDCC | Propanediol Dicaprylate/ Caprate | 8.0 |
| Plantoil Triglyceride | Caprylic/Capric Triglyceride | 4.0 |
| Tocopherol | Tocopherol | 0.05 |
| Demin. Water | Aqua | Ad 100 |
| Glycerin | Glycerin | 2.0 |
| Hydrolite 7 green | 1,2-Heptanediol | 0.6 |
| Methylheptylglycerin | | 0.2 |
| Nativacare® 8600 | Oryza Sativa (Rice) Starch | 1.0 |
| Keltrol ®CG-SFT | Xanthan Gum | 0.4 |
| 3-Hydroxypropyldodecanoate | | 0.1 |
| Sirhamnose | Silanetriol (and) Rhamnose | 3.0 |
| D-Panthenol 75 L | Panthenol | 1.0 |
| Fragrance composition | Parfum | 0.3 |

Leave on w/o

**Table 46:** Baby Nappy Rash Cream w/o

| Ingredients | Amount |
|---|---|
| SymOcide PH<br>Phenoxyethanol, Hydroxyacetophenone, Caprylyl Glycol, Water (Aqua) | 1.0 |
| Cupuaçu butter<br>Theobroma Grandiflorum Seed Butter | 1.0 |
| Cutina HR Powder<br>Hydrogenated Castor Oil | 1.5 |
| Dehymuls PGPH<br>Polyglyceryl-2 Dipolyhydroxystearate | 5.0 |
| Glycerin<br>Glycerin | 5.0 |
| Jojoba oil<br>Simmondsia Chinensis (Jojoba) Seed Oil | 5.0 |
| Magnesium Sulfate Hepta Hydrate<br>Magnesium Sulfate | 0.5 |
| Monomuls 90-O18<br>Glyceryl Oleate | 1.0 |
| Neutral oil<br>Caprylic/capric triglyceride | 8.0 |
| PCL Liquid 100<br>Cetearyl Ethylhexanoate | 5.0 |
| SymCalmin<br>Butylene Glycol, Pentylene Glycol, Hydroxyphenyl Propamidobenzoic Acid | 1.0 |
| Tamanu oil<br>Calophyllum Inophyllum Seed Oil | 0.2 |
| Tetrasodium EDTA<br>Tetrasodium EDTA | 0.1 |
| Titan dioxide<br>Titan dioxide | 4.0 |
| Water<br>Aqua | ad 100 |
| Wheat germ oil<br>Triticum Vulgare (Wheat) Germ Oil | 2.0 |
| Zinc oxide<br>Zinc oxide | 10.0 |
| Fragrance composition<br>Fragrance | 0.1 |
| 3-Hydroxypropyldodecanoate | 0.4 |
| Itaconic Acid | 0.3 |

**Table 47:** Natural Anti-Stretch Mark Cream

| Raw material | INCI | Amount |
|---|---|---|
| Deionised Water | Aqua | Ad 100 |
| Glycerin 85% | Glycerin; Aqua | 7.00 |

(continued)

| Raw material | INCI | Amount |
|---|---|---|
| Zinc Sulfate Heptahydrate | Zinc Sulfate | 1.00 |
| Cristalhyal MW+ | Sodium Hyaluronate | 0.05 |
| Dermofeel ®GO Soft | Polyglyceryl-2-Sesquioleate | 2.50 |
| SymLite® G8 | Glyceryl Caprylate | 0.50 |
| Kahlwax 8104 | Cera Alba | 2.00 |
| Cutina HR Powder | Hydrogenated Castor Oil | 0.50 |
| Dermofeel® sensolv | Isoamyl Laurate | 8.00 |
| Cupuacu Butter Refined | Theobroma Grandiforum Seed Butter; Tocopherol; Hellianthus Annuus Seed Oil | 2.00 |
| Almond Oi, refined, Ph.Eur. | Prunus Amygdalus Dulcis Oil | 2.00 |
| Dermofeel® Toco 70 non GM | Tocopherol, Hellianthus Annuus Seed Oil | 0.20 |
| DL-alpha-Tocopheryl Acetate | Tocopheryl Acetate | 0.50 |
| Magnesium Stearate | Magnesium Stearate | 0.70 |
| 3-Hydroxypropyldecanoate | | 0.30 |
| Phytosqualan, veg. grade | Squalane | 10.00 |
| Hydraprone Nature | Betain, Aqua, Glycerin, Hydrogenated Lecithin, Mel, Glyceryl Caprylate, Potassium Sorbate; Xanthan Gum, Cinnamic Acid, Levulinic Acid, Petctin, Sodium Levulinate, Tocopherol, Sodium Phytate | 2.00 |
| Fragrance composition | Parfume | 0.35 |

SUN CARE

**Table 48:** Anti-Sweat Sunscreen Lotion

| Raw material | INCI | Amount |
|---|---|---|
| Aqua/Water | Aqua | Ad 100 |
| Citric Acid 30% Sol. | Aqua, Citric acid | 0.6 |
| Aristoflex® Velvet | Polyacrylate crosspolymer 11 | 0.6 |
| Glycerin 99.5 % | Glycerin, Aqua | 2.0 |
| SymSol® PF-3 | Aqua, Pentylene glycol, Sodium lauryl sulfoacetate, Sodium oleoyl sarcosinate, Sodium chloride, Sodium oleate | 1.0 |
| 3-Hydroxypropyldecanoate | | 0.4 |
| SymSave® H | Hydroxyacetophenone | 0.3 |
| Neo Heliopan® 357 | Butyl methoxydibenzoylmethane | 3.0 |
| Neo Heliopan® 303 | Octocrylene | 10.0 |
| Neo Heliopan® OS | Ethylhexyl salicylate | 5.0 |
| Neo Heliopan® HMS | Homosalate | 10.0 |
| Neo Heliopan® E1000 | Isoamyl p-methoxycinnamate | 2.0 |
| Neo Heliopan® BMT | Bis-ethylhexyloxyphenol methoxyphenyl triazine | 3.0 |

(continued)

| Raw material | INCI | Amount |
|---|---|---|
| Floraesters K-100® Jojoba | Hydrolyzed jojoba esters<br>Jojoba esters<br>Aqua | 2.0 |
| Dragoxat® 89 | Ethylhexyl isononanoate | 6.0 |
| SymMollient® S green | Cetearyl nonanoate | 1.0 |
| EDTA® BD | Disodium edta | 0.1 |
| SymDecanox™ HA | Caprylic/capric triglyceride<br>Hydroxymethoxyphenyl decanone | 1.0 |
| Frescolat® Plus | Menthol<br>Menthyl lactate | 0.3 |
| Fragrance composition | Parfume | 0.6 |
| SymDeo® Plus | Lauryl alcohol, Phenoxyethanol, 2-benzylheptanol, Decylene glycol | 1.0 |
| Hydroviton® PLUS 2290 | Aqua, Pentylene glycol, Glycerin, Fructose, Urea, Citric acid, Sodium hydroxide, Maltose, Sodium pca, Sodium chloride, Sodium lactate, Trehalose, Allantoin, Sodium hyaluronate, Glucose | 4.0 |
| Sensocel® 10 | Cellulose | 2.0 |

**Table 49:** Anti-Pollution Hair Care (SPF 15)

| Raw material | INCI | Amount |
|---|---|---|
| Caprylic/Capric Triglyceride | Caprylic/capric triglyceride | Ad 100 |
| SymOleo® Vita7 (637260) | Glycine soja oil, Gossypium herbaceum seed oil, Mangifera indica seed butter, Olea europaea fruit oil, Persea gratissima oil, Prunus amygdalus dulcis (sweet almond) oil, Theobroma cacao seed butter | 2.0 |
| SymMollient® PDCC (102119) | Propanediol dicaprylate/caprate | 5.0 |
| Neo Heliopan® BMT 0(102814) | Bis-ethylhexyloxyphenol methoxyphenyl triazine | 2.0 |
| Neo Heliopan® 303 (600154) | Octocrylene | 5.0 |
| Neo Heliopan® OS (131494) | Ethylhexyl salicylate | 5.0 |
| Cetiol® CC | Dicaprylyl carbonate | 5.0 |
| Sweet Almond Oil (659831) | Prunus amygdalus dulcis (sweet almond) oil | 15.0 |
| Cotton Seed Oil (628612) | Gossypium herbaceum seed oil | 15.0 |
| Grape Seed Oil (Refined) (604680) | Vitis vinifera seed oil | 12.0 |
| 3-Hydroxypropyldecanoate | | 0.3 |
| Xiameter® PMX-345 | Cyclopentasiloxane, Cyclohexasiloxane | 10.0 |
| SymHair® Shape & Color (805457) | Cetearyl nonanoate, Triticum vulgare germ oil, Caprylic/capric triglyceride, Linoleic acid, Triticum vulgare bran extract, Triticum vulgare germ extract Camellia oleifera seed oil | 1.0 |
| SymDecanox™ HA (972276) | Caprylic/capric triglyceride, Hydroxymethoxyphenyl decanone | 2.0 |
| Fragrance composition | Parfume | 2.4 |

**Table 50:** Perfect Hand with exp. SPF 15, UVA/UVB Balanced

| Raw material | INCI | Amount |
|---|---|---|
| Emulsiphos® | Potassium cetyl phosphate Hydrogenated palm glycerides | 1.5 |
| Lanette® O | Cetearyl alcohol | 1.0 |
| Tegosoft® MM | Myristyl myristate | 0.5 |
| Neo Heliopan® OS | Ethylhexyl salicylate | 5.0 |
| Edeta® BD | Disodium EDTA | 0.1 |
| Dragoxat® 89 | Ethylhexyl isononanoate | 2.0 |
| Isoadipate | Diisopropyl adipate | 6.0 |
| PCL-Liquid® 100 | Cetearyl ethylhexanoate | 3.0 |
| Corapan® TQ | Diethylhexyl 2,6-naphthalate | 2.0 |
| Cetiol® SB 45 | Butyrospermum parkii butter | 1.5 |
| SymBright™ 2036 | Sclareolide | 0.2 |
| SymDecanox™ HA | Caprylic/capric triglyceride, Hydroxymethoxyphenyl decanone | 1.0 |
| 3-Hydroxypropyldodecanoate | | 0.1 |
| SymCare® O | Hexyldecanol, Pentylene glycol, 4-t-butylcyclohexanol, Bisabolol, | 2.0 |
| | Cetylhydroxyproline palmitamide, Hydroxyphenyl propamidobenzoic acid, Stearic acid, Brassica campestris (rapeseed) sterols, Zingiber officinale (ginger) root extract | |
| SymDetox® | 3,3,5-trimethylcyclohexyl succinate dimethylamide | 0.5 |
| Genuvisco® Carrageenan CG-131 | Chondrus crispus powder | 0.4 |
| Keltrol® CG-SFT | Xanthan gum | 0.2 |
| Aqua/Water | Aqua | Ad 100 |
| Neo Heliopan® Hydro | Phenylbenzimidazole sulfonic acid | 2.5 |
| Biotive® L-Arginine | Arginine | 1.0 |
| Sodium Hydroxide 10% solution | Aqua<br>Sodium hydroxide | 1.3 |
| Glycerin | Glycerin | 2.0 |
| SymSave® H | Hydroxyacetophenone | 0.5 |
| SymDiol® 68 | 1,2-hexanediol, Caprylyl glycol | 0.5 |
| RonaFlair® Flawless | Silica<br>Ci 77892<br>Ci 77491 | 1.5 |
| Extrapone® Moroccan Rose GW | Aqua, Glycerin, Peg-40 hydrogenated castor oil, Rosa damascena flower extract | 1.0 |
| Tego® Feel C10 | Cellulose | 1.5 |
| Fragrance composition | Parfum | 0.5 |

**Table 51:** Daily face cream SPF 20

| Ingredients | INCI | Amount |
|---|---|---|
| SymOcide® PH | Phenoxyethanol, Hydroxyacetophenone, Caprylyl Glycol, Water (Aqua) | 1.0 |
| Ascorbyl Palmitate | Ascorbyl Palmitate | 0.1 |

(continued)

| Ingredients | INCI | Amount |
|---|---|---|
| Biotive L-Arginine | Arginine | 0.2 |
| Buriti oil | Mauritia Flexuosa Fruit Oil | 1.0 |
| Cocoa butter | Theobroma Cacao (Cocoa) Seed Butter | 2.0 |
| Dimethicone | Dimethicone | 0.5 |
| Disodium EDTA | Disodium EDTA | 0.1 |
| Dragosantol 100 | Bisabolol | 0.1 |
| Dragoxat® 89 | Ethylhexyl Isononanoate | 5.0 |
| Emulsiphos | Potassium Cetyl Phosphate, Hydrogenated Palm Glycerides | 2.0 |
| Glycerin | Glycerin | 3.0 |
| Isoadipate | Diisopropyl Adipate | 5.0 |
| Jojoba Wax Flakes | Hydrogenated Jojoba Oil | 1.0 |
| Keltrol CG-T | Xanthan Gum | 0.1 |
| Lanette O | Cetearyl Alcohol | 5.0 |
| Lanette 16 | Cetyl Alcohol | 1.0 |
| Lanette 22 | Behenyl Alcohol | 1.0 |
| Neo Heliopan® 357 | Butyl Methoxydibenzoylmethane | 3.0 |
| Neo Heliopan® HMS | Homosalate | 10.0 |
| Neo Heliopan® Hydro used as a 25% aq, Solution neutralized by arginine | Phenylbenzimidazole Sulfonic Acid | 8.0 |
| 3-Hydroxypropyldodecanoate | | 0.2 |
| Neo Heliopan® OS | Ethylhexyl Salicylate | 5.0 |
| Orgasol Caresse | Polyamide-5 | 1.0 |
| Fragrance composition | Perfum | 0.1 |
| Shea butter | Butyrospermum Parkii (Shea) Butter | 3.0 |
| Simugel EG | Sodium Acrylate / Sodium Acryloyldimethyl Taurate Copolymer, Isohexadecane, Polysorbate 80 | 1.0 |
| SymFinity® 1298 | Echinacea Purpurea Extract | 0.1 |
| 3-Hydroxypropyl benzoate | | 0.8 |
| SymMatrix® | Maltodextrin, Rubus Fructicosus (Blackberry) Leaf Extract | 0.1 |
| SymSitive® 1609 | Pentylene Glycol, 4-t-Butylcyclohexanol | 1.0 |
| Tegosoft TN | C12-15 Alkyl Benzoate | 4.0 |
| Levulinic Acid | Levulinic Acid | 0.2 |
| TEA-salicylate | TEA-salicylate | 0.1 |
| Water | Water (aqua) | ad 100 |

**Table 52:** Sunscreen Oil Spray with Ethanol, exp. SPF 30, UVA/UVB balanced

| Raw material | INCI | Amount |
|---|---|---|
| Neo Heliopan® 357 (622501) | Butyl methoxydibenzoylmethane | 5.0 |
| Neo Heliopan® BMT(102814) | Bis-ethylhexyloxyphenol methoxyphenyl triazine | 3.5 |

(continued)

| Raw material | INCI | Amount |
|---|---|---|
| Neo Heliopan® OS (131494) | Ethylhexyl salicylate | 5.0 |
| Uvinul® T 150 | Ethylhexyl triazone | 5.0 |
| SymMollient® PDCC (102119) | Propanediol dicaprylate/caprate | 5.0 |
| Cetiol® Sensoft | Propylheptyl caprylate | 6.0 |
| Cetiol® AB | C12-15 alkyl benzoate | 30.0 |
| Isopropyl Palmitate | Isopropyl palmitate | 20.7 |
| 3-Hydroxypropyldodecanoate | | 0.1 |
| SymDecanox™ HA (972276) | Caprylic/capric triglyceride Hydroxymethoxyphenyl decanone | 1.0 |
| Ethanol | Alcohol, Aqua | 15.0 |
| Hydrolite® 5 green (996442) | Pentylene glycol | 3.0 |
| Fragrance composition | Parfume | 0.3 |

**Table 53:** Face Protecting Watery Mousse exp. SPF 30*

| Ingredients | INCI | Amount |
|---|---|---|
| Aqua/Water | Aqua | Ad 100 |
| Neo Heliopan® Hydro (103089) | Phenylbenzimidazole sulfonic acid | 2.0 |
| Neo Heliopan® AP (106796) | Disodium phenyl dibenzimidazole tetrasulfonate | 1.0 |
| Biotive® L-Arginine (621277) | Arginine | 1.8 |
| SymSol® PF-3 (358712) | Aqua, Pentylene glycol, Sodium lauryl, sulfoacetate, Sodium oleoyl sarcosinate, Sodium chloride, Sodium oleate | 2.5 |
| 3-Hydroxypropyldecanoate | | 0.4 |
| Dragosine® (844033) | Carnosine | 0.2 |
| Aqua Keep 10SH-NFC | Sodium acrylates crosspolymer-2 | 2.5 |
| SymSave® H (979940) | Hydroxyacetophenone | 0.5 |
| Phenoxyethanol | Phenoxyethanol | 0.5 |
| Hydrolite® 5 (616751) | Pentylene glycol | 1.5 |
| Neo Heliopan® 357 (622501) | Butyl methoxydibenzoylmethane | 3.0 |
| Neo Heliopan® OS (131494) | Ethylhexyl salicylate | 5.0 |
| Neo Heliopan® BMT(102814) | Bis-ethylhexyloxyphenol methoxyphenyl triazine | 3.0 |
| Uvasorb® HEB | Diethylhexyl butamido triazone | 3.0 |
| SymMollient® S (181598) | Cetearyl nonanoate | 3.0 |
| EDTA® BD | Disodium edta | 0.1 |
| Isoadipate (660014) | Diisopropyl adipate | 4.0 |
| Cetiol® AB | C12-15 alkyl benzoate | 7.0 |
| Caprylic/Capric Triglyceride | Caprylic/capric triglyceride | 6.0 |
| Frescolat® ML (620105) | Menthyl lactate | 2.0 |
| SymGlucan® (151719) | Aqua, Glycerin, 1,2-hexanediol, Caprylyl glycol, Beta-glucan | 2.0 |
| Color | - | 0.2 |

**Table 54:** Sunscreen Spray (O/W), exp. SPF 30* (all in one pot / cold manufacturing process)

| Ingredients | INCI | Amount |
|---|---|---|
| Dracorin® GOC | Glyceryl Oleate Citrate Caprylic/ Capric Triglyceride | 2.0 |
| Dragosine® | Carnosine | 0.2 |
| Neo Heliopan® Flat | Homosalate, Octocrylene, Bis-Ethylhexyloxyphenol Methoxyphenyl Triazine, Butyl Methoxydibenzoylmethane Ethylhexyl Salicylate | 25.0 |
| SymMollient® PDCC | Propanediol Dicaprylate/Caprate | 7.0 |
| Aristoflex® Silk | Sodium Polyacryloyldiemthyl taurate | 0.6 |
| Aqua /Water | Aqua | Ad 100 |
| Citric Acid 10% Sol. | Aqua, Citric acid | 0.25 |
| SymOcide® PH | Phenoxyethanol, Hydroxyacetophenone, Caprylyl Glycol, Aqua | 1.45 |
| 3-Hydroxypropyldodecanoate | | 0.8 |
| SymMollient® PDCC (102119) | Propanediol Dicaprylate/ Caprate | 3.0 |
| Glycerin 99.5 % | Glycerin | 3.0 |
| Dragoxat® 89 | Ethylhexyl Isononanoate | 2.0 |
| PCL-Liquid® 100 | Cetearyl Ethylhexanoate | 2.0 |
| Edta® BD | Disodium EDTA | 0.1 |
| Farmal® MS 6135 | Calcium Starch Octenylsuccinate | 2.0 |
| Keltrol® CG-BT | Xanthan Gum | 0.4 |
| Fragrance composition | Parfume | 0.3 |

**Table 55:** Full protection drops expected SPF 50

| Ingredients | INCI | Amount |
|---|---|---|
| Dracorin® GOC | Glyceryl oleate citrate, Caprylic/capric triglyceride | 2.5 |
| Neo Heliopan® 303 | Octocrylene | 8.0 |
| Neo Heliopan® 357 | Butyl methoxydibenzoylmethane | 4.5 |
| Neo Heliopan® HMS | Homosalate | 10.0 |
| Neo Heliopan® OS | Ethylhexyl salicylate | 5.0 |
| Neo Heliopan® BMT | Bis-ethylhexyloxyphenol methoxyphenyl triazine | 5.0 |
| SymMollient® PDCC | Propanediol dicaprylate/caprate | 5.0 |
| Velvesil® DM | Dimethicone, Cetearyl dimethicone crosspolymer | 3.0 |
| Dow Corning® 9041 Silicone Elastomer Blend | Dimethicone, Dimethicone crosspolymer | 2.0 |
| EDTA® BD | Disodium edta | 0.1 |
| Keltrol® CG-SFT | Xanthan gum | 0.2 |
| Pemulen™ TR-2 Polymeric Emulsifier | Acrylates/c10-30 alkyl acrylate crosspolymer | 0.1 |
| Aqua/Water | Aqua | Ad 100 |
| Glycerin 99.5 % | Glycerin, Aqua | 3.0 |
| Dragosine® | Carnosine | 0.2 |
| SymSave® H | Hydroxyacetophenone | 0.5 |
| Hydrolite 7 green | 1,2-Heptaned iol | 0.5 |
| Hydrolite® CG | Caprylyl glycol | 0.3 |
| SymVital® AR (974839) | Zingiber officinale (ginger) root extract | 0.2 |

(continued)

| Ingredients | INCI | Amount |
|---|---|---|
| 3-Hydroxypropyldecanoate | | 0.4 |
| Tapioca Pure | Tapioca starch | 2.0 |
| Fragrance composition | Parfume | 0.5 |

**Table 56:** Urban Sun Spray exp. SPF 50+

| Ingredients | INCI | Amount |
|---|---|---|
| Aqua /Water | Aqua | Ad 100 |
| Vivapur® CS Tex sun | Microcrystalline Cellulose, Cellulose Gum | 1.0 |
| Vivapur® CS 032 XV | Microcrystalline Cellulose, Xanthan Gum | 1.0 |
| SymOcide® PH | Phenoxyethanol, Hydroxyacetophenone, Caprylyl Glycol, Aqua | 1.4 |
| Emulsiphos® | Potassium cetyl phosphate, Hydrogenatedpalm glycerides | 2.5 |
| Dragoxat®89 | Ethylhexyl Isononanoate | 2.0 |
| SymMollient ® PDCC | Propanediol Dicaprylate/Caprate | 3.5 |
| SymSol® PF-3 | Aqua, Pentylene Glycol, Sodium Lauryl Sulfoacetate, Sodium Oleoyl Sarcosinate, Sodium Chlrodie, Sodium Oleate | 2.0 |
| Prisorine™ 3505 | Isostearic acid | 1.5 |
| Neo Heliopan® BMT | Bis-Ethylhexyloxyphenol Methoxypenyl Triazine | 2.5 |
| Neo Heliopan® 357 | Butyl Methoxydibenzoylmethane | 4.5 |
| Neo Heliopan® 303 | Octocrylene | 10.0 |
| Neo Heliopan® HMS | Homosalate | 10.0 |
| Neo Heliopan® OS | Ethylhexyl Salicylate | 5.0 |
| Dragosine® | Carnosine | 0.2 |
| SymUrban® | Benzylidene Dimethoxydimetylindanone | 0.2 |
| SymVital® AR 3040 | Zingiber officinale (Ginger) Root extract | 0.1 |
| 3-Hydroxypropyldecanoate | | 0.25 |
| SymDecanox™ HA | Caprylic/Capric Triglyceride, Hydroxymethoxyphenyl decanone | 1.0 |
| Floraesters K-100® Jojoba | Hydrolyzed jojoba esters, Jojoba esters, Aqua | 0.5 |
| Benzyl salicylate | | 0.1 |
| EDTA BD | Disodium EDTA | 0.1 |
| Keltrol® CG-BT | Xanthan Gum | 0.3 |
| Fragrance composition | Parfume | 0.5 |
| Farmal® MS6135 | Calcium Starch Octenylsuccinate | 2.0 |
| Tapioca Pure | Tapioca starch | 1.5 |

WIPES

**Table 57:** Clear Anti Acne Wipe

| Ingredients | INCI | Amount |
|---|---|---|
| Farnesol | Farnesol | 0.2 |

(continued)

| Ingredients | INCI | Amount |
|---|---|---|
| Methylisothiazolinone | Methylisothiazolinone | 0.1 |
| Isopropanol | Isopropanol | 45.0 |
| Water, distilled | Water (Aqua) | Ad 100 |
| Camomile CL | Chamomilla Recutita (Matricaria) Flower Water, Butylene Glycol, Pentylene Glycol | 1.0 |
| Witch Hazel-Distillate | Hamamelis Virginiana (Witch Hazel) Water, Water (Aqua), Alcohol, Hamamelis Virginiana (Witch Hazel) Extract | 1.0 |
| 3-Hydroxypropyldecanoate | | 0.4 |
| Citric Acid 10 % aqueous solution | Citric Acid | 0.03 |
| Fragrance composition | Parfume | 0.1 |
| Hydroxyethoxyphenyl Butanone | Hydroxyethoxyphenyl Butanone | 0.4 |

**Table 58:** Intimate Wipes

| Ingredients | INCI | Amount |
|---|---|---|
| Dracorin® GOC | Glyceryl Oleate Citrate, Caprylic/Capric Triglyceride | 2.2 |
| PCL-Liquid® 100 | Cetearyl Ethylhexanoate | 3.0 |
| SymCalmin® | Pentylene Glycol, Butylene Glycol, Hydroxyphenyl Propamidobenzoic Acid | 1.0 |
| Frescolat® X-Cool | Menthyl Ethylamido Oxalate | 0.5 |
| Fragrance composition | Parfume | 0.2 |
| Water, distilled | Water (Aqua) | Ad 100 |
| PemulenTM TR-2 Polymeric Emulsifier | Acrylates/C10-30 Alkyl Acrylate Crosspolymer | 0.2 |
| SymOcide® PH | Phenoxyethanol, Hydroxyacetophenone, Caprylyl Glycol, Aqua | 1.3 |
| 3-Hydroxypropyldodecanoate | | 0.7 |
| Potassium Sorbate | Potassium Sorbate | 0.1 |
| Extrapone® Camomille GW | Glycerin,Aqua, Chamomilla, Recutita Flower Extract | 0.5 |
| Sodium Hydroxide 10% solution | Aqua, Sodium Hydroxide | 0.45 |
| PCA ethyl cocoyl arginate | PCA ethyl cocoyl arginate | 0.1 |

MASKS

**Table 59:** Warming Mineral Mask

| Ingredients | EU INCI | Amount |
|---|---|---|
| Glycerin | Glycerin | 3.0 |
| Keltrol® CG-SFT | Xanthan Gum | 0.3 |
| Aqua/Water | Aqua | Ad 100 |
| Edeta BD | Disodium EDTA | 0.1 |
| Kaolin | Kaolin | 10.0 |
| Bentonite | Bentonite | 5.0 |

(continued)

| Ingredients | EU INCI | Amount |
|---|---|---|
| Dracorin® CE | Glyceryl Stearate Citrate | 2.0 |
| Lanette® O | Cetearyl Alcohol | 1.7 |
| Brij™ 72 | Steareth-2 | 0.8 |
| SymMollient® S green | Cetearyl Nonanoate | 1.0 |
| 3-Hydroxypropyldodecanoate | | 1.2 |
| Mango Butter | Mangifera Indica Seed Butter | 0.8 |
| Alpha Tocopherol DL | Tocopherol | 0.2 |
| SymDecanox™HA | Caprylic/Capric Trigylceride, Hydroxymethoxyphenyl Decanone | 0.1 |
| SymClariol® | Decylene Glycol | 0.25 |
| SymSave® H | Hydroxyacetophenone | 0.5 |
| Hydrolite® 5 green | Pentylene Glycol | 4.0 |
| Citric Acid, 30% solution | Aqua, Citric Acid | 0.07 |
| SymVital® AR 3040 | Zingiber officinale (Ginger) Root Extract | 0.15 |
| Thermolat® | Vanillyl Butyl Ether, 1,2-Hexanediol, Capryly Glycol | 0.3 |
| Hydroviton® PLUS 2290 | Aqua, Pentylene Glycol, Glycerin, Fructose, Urea, Citric acid, Sodium hydroxide, Maltose, Sodium PCA, Sodium Chloride, Sodium Lactate, Trehalose, Allantoin, Sodium hyaluronate, Glucose | 2.0 |
| Fragrance composition | Parfum | 0.3 |
| Extrapone Charcol® G CA | Glycerin, Charcoal Powder | 5.0 |
| Extrapone® Coconut Water GW | Aqua, Glycerin, Cocos Nucifera fruit Juice | 10.0 |
| Colour I | | 0.8 |
| Colour II | | 4.0 |

CONCENTRATES & SOLID FORMULATIONS

**Table 60:** Energy Concentrate

| Ingredients | EU INCI | Amount |
|---|---|---|
| Aqua/Water | Aqua | Ad 100 |
| Pemulen™ TR-2 Polymeric Emulsifier | Acrylates/C10-30Alkyl Acrylate Crosspolymer | 0.5 |
| Glycerin 99.5% | Glycerin | 2.0 |
| SymVital® AR 3040 | Zinbiber officinale (ginger) root extract | 0.1 |
| SymFinity® 1298 | Echinacea purpurea extract | 0.5 |
| Hydrovition® PLUS 2290 | Aqua, Pentylene Glycol, Glycerin, Fructose, Urea, Citric acid, Sodium hydroxide, Maltose, Sodium PCA, Sodium Chloride, Sodium Lactate, Trehalose, Allantoin, Sodium hyaluronate, Glucose | 1.0 |
| Extrapone® Orange (Organic) GW | Aqua,Glycerin, Citrus aurantium dulcis juice | 1.0 |
| Actipone® Black Coffee GW | Aqua, Coffea Arabica Seed Extract, Coffea Robusta Seed Extract | 0.5 |
| SymSol® PF-3 | Aqua, Pentylene Glycol, Sodium Lauryl Sulfoacetate, Sodium Oleoyl Sarcosinate, Sodium Chloride, Sodium Oleate | 3.0 |

(continued)

| Ingredients | EU INCI | Amount |
|---|---|---|
| 3-Hydroxypropyldecanoate | | 1.0 |
| SymRepair® 100 | Hexyldecanol, Bisabolol, Cethylhydroxyproline Palmitamide, Stearic acid, Brassica Campestris (Rapeseed) sterols | 0.2 |
| Tocopherol Alpha DL | Tocopherol | 0.2 |
| Hydrolite® 5 green | Pentylene Glycol | 5.0 |
| Fragrance composition | Parfum | 0.1 |
| Sodium Hydroxide, 10% solution | Aqua, Sodium hydroxide | 1.5 |

**Table 61:** Beard Tamer

| Ingredients | EU INCI | Amount |
|---|---|---|
| Eutanol® G | Octyldodecanol | 1.0 |
| GP-1 | Dibutyl Lauroyl Glutamide | 2.4 |
| EB-21 | Dibutyl Ethylhexanoyl Glutamide | 1.6 |
| Dragoxat® 89 | Ethylhexyl Isononanoate | 14.0 |
| SymTriol® | Caprylyl Glycol, 1,2-Hexanediol, Methylbenzyl alcohol | 0.3 |
| Sweet Almond Oil (Organic) | Prunus Amygdalus Dulcis (Sweet Almond) Oil | Ad 100 |
| 3-Hydroxypropyldodecanoate | | 1.5 |
| Jojoba Oil (Organic) | Simmodsa Chinenis Seed Oil | 10.0 |
| SymRelief® 100 | Bisabolol, Zingiber Officinale Root Extract | 0.1 |
| Hydrolite® 5 green | Pentylene Glycol | 2.0 |
| SymLite® G8 | Glyceryl Caprylate | 0.5 |
| SymMollient® PDCC | Propanediol Dicaprylate/Caprate | 10.0 |
| Moringa Oil CA | Moringa Oleifera Seed Oil | 1.0 |
| SymHair® Shape & Color | Cetearyl Nonanoate, Triticum Vulgare Germ Oil, Caprylic/Capric Triglyceride, Linoleic Acid, Triticum Vulgre Bran Extract, Triticum Vulgare Germ Extract, Camellia Oleifera Seed Oil | 0.5 |
| SymDecanox™ HA | Caprylic/Capric Triglyceride, Hydroxymethoxyphenyl Decanone | 1.5 |
| Fragrance composition | Parfum | 1.0 |

**Table 62:** Sensi-SCALP (Green Solid Shampoo)

| Ingredients | EU INCI | Amount |
|---|---|---|
| AMISOFT® MS-11 | Sodium Myristoyl Glutamate | 30.0 |
| ELFAN® AT 84 | Sodium cocoyl Isethionate | 15.0 |
| ImerCare® 02K-S | Kaolin | 18.8 |
| Hydrolite® 5 green | Pentylene Glycol | 1.5 |
| Dragosantol® 100 | Bisabolol | 0.1 |
| Potassium Sorbate | Potassium Sorbate | 0.3 |
| Aqua/Water | Aqua | Ad 100 |
| SymDecanox™ HA | Caprylic/Capric Triglyceride, Hydroxymethoxyphenyl decanone | 1.5 |
| Alpha Tocopherol DL | Tocopherol | 0.5 |

(continued)

| Ingredients | EU INCI | Amount |
|---|---|---|
| 3-Hydroxypropyldecanoate | | 1.1 |
| Cetiol® SB45 | Butyrospermum parkii butter | 13.0 |
| Extrapone® Rooibus GW | Aqua, Glycerin, Aspalathus Linearis leaf extract | 1.0 |
| SymReboot™ L19 | Maltodextrin, Lactobacillus Ferment | 0.5 |
| SymOleo® Vita7 | Glycine soja oil, Gossypium herbaceum seed oil, mangifera idica seed butter, olea europaea fruitoil, persea gratissima oil, prununs amygdalus dulcis (sweet almond) oil, Theobroma cacao seed butter | 1.0 |
| Fragrance composition | Parfum | 1.2 |

**Table 63:** Hair Soap SHAMPOO Bar

| Ingredients | EU INCI | Amount |
|---|---|---|
| Talco | Talc | Ad 100 |
| Vivapur® CS 032 XV | Microcrystaline Cellulose, Xanthan Gum | 0.3 |
| Texapon® ZACD | Sodium Lauryl Sulfate | 10.00 |
| SymOleo® Vita7 | Glycine soja oil, Gossypium herbaceum seed oil, mangifera idica seed butter, olea europaea fruitoil, persea gratissima oil, prununs amygdalus dulcis (sweet almond) oil, Theobroma cacao seed butter | 2.00 |
| 3-Hydroxypropyldecanoate | | 0.75 |
| Cetiol® SB 45 | Butyrospermum parkii butter | 3.00 |
| Mackamide® CMA | Cocamide Mea | 4.00 |
| SymSol® PF-3 | Aqua, Pentylene Glycol, Sodium Lauryl sulfoacetate, sodium oleoyl sarcosinate, Sodium Chloride, Sodium Oleate | 5.00 |
| Food Color Beta Carotene E160A OilSoluble | Helianthus annuus (Sunflower) seed oil, Beta-Carotene (CI40800) | 0.45 |
| SymOcide® PH | Phenoxyethanol, Hydroxyacetophenone, Caprylyl Glycol, Aqua | 1.00 |
| SymHair® Restore | Glycerin, Triticum vulgare protein, Aqua | 1.00 |
| Fragrance composition | Parfum | 1.50 |

GLASSCLEANERS

**Table 64:** Glass cleaner

| **Name** | **Amount** |
|---|---|
| Aqua | Ad 100 |
| Alcohol denat | 5.0 |
| Propyleneglycolbutylether | 1.0 |
| Lauryldimethylamineoxide | 0.5 |
| 3-Hydroxypropyldodecanoate | 0.1 |
| Sodium Hydroxide | 0.2 |
| Denatonium Benzoate | 0.1 |

**Table 65:** Glass cleaner

| **Name** | **Amount** |
|---|---|
| Aqua | Ad 100 |
| 3-Hydroxypropyldecanoate | 0.3 |
| Alcohol denat | 4.5 |
| Sodium C13-17 Alkane Sulfonate/ SLS | 0.2 |
| Denatonium Benzoate | 0.02 |
| Butoxypropanol | 0.5 |

ALL PURPOSE AND FLOOR CLEANERS

**Table 66:**

| Ingredients | Amount |
|---|---|
| Fatty alcohol ethoxylated | 4.4 |
| Sulfonic acid, C 13-17-sec-Alkyl-, Sodium salt | 3.6 |
| Methylisothiazolinone | 0.01 |
| Fragrance | 0.9 |
| 3-Hydroxypropyldodecanoate | 0.6 |
| Protease (Subtilisin) | 0.7 |
| Amylase | 0.4 |
| Aqua | ad 100 |

**Table 67:**

| Ingredients | Amount |
|---|---|
| Coco-Glycoside | 1 |
| Benzalkonium Chloride | 0.2 |
| Sodium bicarbonate | 0.1 |
| Tri-sodiumcitrate-dihydrate | 0.1 |
| Methylglycinediacetic acid | 0.1 |
| 3-Hydroxypropyldecanoate | 0.5 |
| 1,2-Benzisothiazol-3(2H)-on | 0.1 |
| Fragrance | 0.1 |
| 1,2-heptanediol | 0.5 |
| Amylase | 0.3 |
| Aqua | ad 100 |

**Table 68:** High Gloss Floor Cleaner pH 8

| Ingredient | Amount |
|---|---|
| Rhamnolipid | 1.0 |
| Sorbitan ester surfactant | 0.5 |
| Sodium C14-16 Olefin Sulfonate | 4.0 |

(continued)

| Ingredient | Amount |
|---|---|
| 3-Hydroxypropyldodecanoate | 0.7 |
| Water | Ad 100 |
| Cocamidopropylbetaine | 1.0 |
| PE Wax | 0.5 |
| Preservative | q.s. |

KITCHEN CLEANERS

**Table 69:** Cleaner against fat and burned in

| Ingredient | Amount |
|---|---|
| Aqua | Ad 100 |
| MEA / Monoethanolamine / Ethanolamine | 3.0 |
| 3-Hydroxypropyldecanoate | 1.0 |
| Butoxypropanol | 1.0 |
| Sodium Etidronate / EHDP | 1.0 |
| Lauryldimethylamineoxide | 0.55 |
| Alcohols C16-18 and C18 Unsatd. Ethoxylated 5 EO | 0.5 |
| Sodium Carbonate | 0.5 |
| Etidronic Acid | 0.5 |
| Perfume | 0.2 |
| 2-t-butylcyclohexyl acetate | 0.01 |
| Phenethyl alcohol | 0.01 |
| Hexahydro-methanoidenyl propionate | 0.01 |

**Table 70:** Grapefruit Kitchen Cleaner

| Ingredient | Amount |
|---|---|
| Aqua | Ad 100 |
| Sodium Carbonate | 1.0 |
| Perfume | 0.2 |
| Phenoxyisopropanol | 5.0 |
| Tetrasodium Glutamate Diacetate | 0.5 |
| 3-Hydroxypropyldodecanoate | 0.5 |
| Sodium Laureth Sulfate | 2.5 |
| Olive Oil Fatty Acid | 1.0 |
| Methyl Diethanolamine | 2.0 |
| Triethnolamine | 1.0 |
| Amide Polyglycol Ether | 0.5 |
| Glycerine | 0.2 |

HAND DISH WASH LIQUIDS (HDWL)

**Table 71:** Dishwash liquid manual

| Ingredient | Amount |
|---|---|
| Sodium Laureth Sulfate (37%) | Ad 100 |
| Cocoamidopropylbetaine | 10.0 |
| Ethanol | 1.0 |
| Benzisothiazolinone, Methylisothiazolinone, Laurlyamine, Dipropylenediamine | 0.1 |
| 3-Hydroxypropyldecanoate | 0.7 |
| Sodium Chloride | 0.6 |
| Perfume | 0.4 |
| 1,2-Propanediol | 5.0 |
| Sodium Hydroxyide (30% sol) | 0.16 |

**Table 72:** HDWL

| Ingredient | Amount |
|---|---|
| Aqua | Ad 100 |
| Sodium Laureth Sulfate | 7.5 |
| Alpha Olefin Sulfonate 7 | 7.5 |
| Cocamidopropylamineoxide | 2.5 |
| Cocamidopropylbetaine | 2.5 |
| C10 Pareth-8 | 2.5 |
| Sodium Chloride | 1.0 |
| Sodium Xylene Sulfonate | 1.0 |
| 3-Hydroxypropyldecanoate | 0.9 |
| Perfumes | 0.2 |
| Citric Acid | 0.2 |
| Polyethylenimine ethoxylated | 0.2 |
| CMIT/ MIT/ BIT | 0.05 |

LIQUID DETERGENTS

**Table 73:** Detergent liquid light duty

| Ingredients | Amount |
|---|---|
| Cocos fatty acid | 0.85 |
| Potassium hydroxide | 0.45 |
| 3-Hydroxypropyldodecanoate | 0.35 |
| 2-octyl-2H-isothiazol-3-one | 0.2 |
| Sulfonic acid, C 13-17-sec-Alkyl-, Sodium salt | 20.0 |
| Sodium Laureth Sulfate | 3.0 |

(continued)

| Ingredients | Amount |
|---|---|
| Trideceth-9 | 5.0 |
| Sodium Chloride | 1.3 |
| Fragrance | 0.5 |
| Protease (Subtilisin) | 0.6 |
| Amylase | 0.3 |
| Aqua | ad 100 |

**Table 74:** Color detergent

| Ingredient | Amount |
|---|---|
| Aqua | 60.0 |
| Sodium Dodecylbenzenesulfonate | 0.2 |
| MEA Dodeylbenzensulfonate | 9.0 |
| C12-18 Fatty Alcohol 7 EO | 9.0 |
| Propylene Glycol | 8.0 |
| 3-Hydroxypropyldecanoate | 0.7 |
| MEA Citrate | 4.0 |
| Glycerine | 1.0 |
| Sodium Etidronate | 1.0 |
| Sodium Metaborate | 0.5 |

**Table 75:** Liquid detergent

| Ingredient | Amount |
|---|---|
| Aqua | Ad 100 |
| MEA Dodeylbenzenesulfonate | 12.0 |
| Alcohols C12-18 Ethoxylated | 6.0 |
| Perfumes | 0.2 |
| Propylene Glycol | 2.0 |
| Diethylenetriamine Pentamethylphosphonate | 0.4 |
| Alcohol Denat | 2.0 |
| 3-Hydroxypropyldodecanoate | 0.1 |
| MEA Citrate | 1.0 |
| Sodium Borate | 1.0 |
| MEA Palm kernelate | 1.0 |
| Sodium Chloride | 0.2 |
| Phenoxyethanol | 0.2 |

## Claims

1. Use of a compound of formula (I)

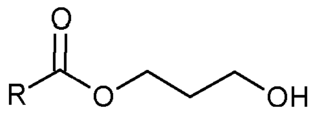

(I)

wherein R is selected from the group consisting of n-nonyl and n-undecyl,
as an antimicrobial agent

(i) for inhibiting or preventing the growth of one or more microorganisms in a personal care, household, or pharmaceutical product, and/or
(ii) for inhibiting or preventing the growth of one or more microorganisms on a surface.

2. Non-therapeutic use of a compound of formula (I) as defined in claim 1 as an antimicrobial agent for inhibiting or preventing the growth of one or more microorganisms on a mammal's, preferably human's, skin or mucosa, preferably skin, more preferably human axillary skin,
most preferably use of a compound of formula (I) as defined in claim 1 as a deodorant active.

3. Use according to claim 1 or non-therapeutic use according to claim 2, wherein the one or more microorganisms is/are selected from the group consisting of *Epidermophyton floccosum, Trichophyton rubrum, Staphylococcus epidermidis, Staphylococcus haemolyticus, Staphylococcus hominis, Micrococcus luteus, Acinetobacter haemolyticus, Aspergillus fumigatus, Streptococcus mutans, Porphyromonas gingivalis, Fusobacterium nucleatum, Prevotella intermedia, Malassezia furfur, Malassezia globosa, Malassezia restricta, Malassezia sympodialis, Staphylococcus capitis, Gardnerella vaginalis, Streptococcus agalactiae, Fannyhessea vaginae, Peptoniphilus lacrimalis, Cutibacterium acnes, Staphylococcus aureus, Candida albicans, Aspergillus brasiliensis, Pseudomonas aeruginosa, Escherichia coli, Salmonella enteritidis, Corynebacterium xerosis, Corynebacterium jeikeium, Anaerococcus octavius, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Streptococcus pyogenes, Streptococcus pneumonia, Moraxella osloensis, Kokuria oxytoca, Kokuria pneumonia, Kokuria palustris, Kokuria rhizophilia, Sphingobium yanoikuyae, Stenotrophomonas maltophilia, Pseudomonas cremoricolorata, Bacillus subtilis, Enterobacter gergoviae, Trichophyton mentagrophytes, Brevibacterium epidermidis, Klebsiella pneumonia, Pseudomonas fluorescens, Pseudomonas putida, Penicillium funiculosum, Bacillus licheniformis,* and *Enterococcus hirae.*

4. Use according to claim 1 or 3,

wherein the personal care product is selected from the group consisting of deodorant products, body care and cleansing products, intimate care and cleansing products, foot care and cleansing products, oral care and cleansing products, scalp and hair care and cleansing products, and face care and cleansing products, more preferably wherein the personal care product is selected from the group consisting of deodorant sprays, deodorant aerosols, deodorant roll-ons, deodorant sticks, deodorant creams, foot creams, foot powders, body washes, mouth washes, toothpastes, shampoos, hair conditioner, styling wax, styling gels, styling foams, styling creams, styling mousses, styling serums, styling pomades, micellar waters, intimate washes, cleansers, toners, serums, moisturizers, eye creams, balms, soaps, facial masks, sunscreens, liquids for wet wipes, primers, concealers, foundations, rouges, bronzers, highlighters, eyebrow pencils, eyebrow creams, eyebrow waxes, eyebrow gels, eyebrow powders, eyeshadows, eyeliners, mascara, lipsticks, lip glosses, lip liners, lip balms, face powders, setting powders, setting sprays, and nail polishes,
and/or
wherein the household product is selected from the group consisting of alcohol free glass cleaners, glass cleaners, all-purpose cleaners, all-purpose sprays, degreasers, carpet cleaners, wood cleaners, floor cleaners, laminate cleaners, vinyl cleaners, ceramic cleaners, cork cleaners, linoleum cleaners, porcelain cleaners, stone cleaners, concrete cleaners, parquet cleaners, high gloss floor cleaners, kitchen cleaners, fat solver cleaners, bathroom cleaners, toilet cleaners, chalk cleaners, automatic dish washes, hand dish wash fluids, antibacterial hand dish wash fluids, fabric softeners, fabric care, liquid laundry detergents, powder laundry detergents, and hygiene wash detergents,
and/or
wherein the pharmaceutical product is selected from the group consisting of deodorant sprays, deodorant roll-ons, deodorant sticks, deodorant creams, foot creams, body washes, mouth washes, toothpastes, shampoos,

intimate washes, cleansers, serums, moisturizers, eye creams, balms, soaps, facial masks, sunscreens, and liquids for wet wipes.

**5.** Use according to claim 1 or 3, wherein the surface is a solid surface, preferably selected from the group consisting of kitchen surfaces, bathroom surfaces, drums of washing machines, cutlery, crockery, wood flooring (solid wood flooring, engineered wood flooring), laminate, vinyl, ceramic, cork, linoleum, porcelain, stone, concrete, and parquet, or is a non-solid surface, preferably selected from the group consisting of woven materials and leathers, more preferably fabrics, carpets, tissues, clothes, sheets, and sponges.

**6.** Method

(i) for inhibiting or preventing the growth of one or more microorganisms in a personal care, household, or pharmaceutical product, and/or
(ii) for inhibiting or preventing the growth of one or more microorganisms on/in a surface,

comprising or consisting of the following steps:

(a) Providing a compound of formula (I) as defined in claim 1, and
(b) bringing the compound provided in step (a)

(i) in contact with the other ingredients of a personal care, household, or pharmaceutical product, in which the growth of one or more microorganisms is to be inhibited or prevented, and/or
(ii) in contact with a surface, on/in which the growth of one or more microorganisms is to be inhibited or prevented.

**7.** Method according to claim 6, wherein the one or more microorganisms, whose growth is inhibited or prevented, is/are selected from the group consisting of *Epidermophyton floccosum, Trichophyton rubrum, Staphylococcus epidermidis, Staphylococcus haemolyticus, Staphylococcus hominis, Micrococcus luteus, Acinetobacter haemolyticus, Aspergillus fumigatus, Streptococcus mutans, Porphyromonas gingivalis, Fusobacterium nucleatum, Prevotella intermedia, Malassezia furfur, Malassezia globosa, Malassezia restricta, Malassezia sympodialis, Staphylococcus capitis, Gardnerella vaginalis, Streptococcus agalactiae, Fannyhessea vaginae, Peptoniphilus lacrimalis, Cutibacterium acnes, Staphylococcus aureus, Candida albicans, Aspergillus brasiliensis, Pseudomonas aeruginosa, Escherichia coli, Salmonella enteritidis, Corynebacterium xerosis, Corynebacterium jeikeium, Anaerococcus octavius, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Streptococcus pyogenes, Streptococcus pneumonia, Moraxella osloensis, Kokuria oxytoca, Kokuria pneumonia, Kokuria palustris, Kokuria rhizophilia, Sphingobium yanoikuyae, Stenotrophomonas maltophilia, Pseudomonas cremoricolorata, Bacillus subtilis, Enterobacter gergoviae, Trichophyton mentagrophytes, Brevibacterium epidermidis, Klebsiella pneumonia, Pseudomonas fluorescens, Pseudomonas putida, Penicillium funiculosum, Bacillus licheniformis,* and *Enterococcus hirae.*

**8.** Method according to claim 6 or 7,

wherein the personal care product is selected from the group consisting of deodorant products, body care and cleansing products, intimate care and cleansing products, foot care and cleansing products, oral care and cleansing products, scalp care and cleansing products, and face care and cleansing products, more preferably wherein the personal care product is selected from the group consisting of deodorant sprays, deodorant roll-ons, deodorant sticks, deodorant creams, foot creams, body washes, mouth washes, toothpastes, shampoos, hair conditioner, styling wax, styling gels, styling foams, styling creams, styling mousses, styling serums, styling pomades, micellar waters, intimate washes, cleansers, toners, serums, moisturizers, eye creams, balms, soaps, facial masks, sunscreens, liquids for wet wipes, primers, concealers, foundations, rouges, bronzers, highlighters, eyebrow pencils, eyebrow creams, eyebrow waxes, eyebrow gels, eyebrow powders, eyeshadows, eyeliners, mascara, lipsticks, lip glosses, lip liners, lip balms, face powders, setting powders, setting sprays, and nail polishes,
and/or
wherein the household product is selected from the group consisting of alcohol free glass cleaners, glass cleaners, all-purpose cleaners, all-purpose sprays, degreasers, carpet cleaners, wood cleaners, floor cleaners, laminate cleaners, vinyl cleaners, ceramic cleaners, cork cleaners, linoleum cleaners, porcelain cleaners, stone

cleaners, concrete cleaners, parquet cleaners, high gloss floor cleaners, kitchen cleaners, fat solver cleaners, bathroom cleaners, toilet cleaners, chalk cleaners, automatic dish washes, hand dish wash fluids, antibacterial hand dish wash fluids, fabric softeners, fabric care, liquid laundry detergents, powder laundry detergents, and hygiene wash detergents,
and/or
wherein the pharmaceutical product is selected from the group consisting of deodorant sprays, deodorant roll-ons, deodorant sticks, deodorant creams, foot creams, body washes, mouth washes, toothpastes, shampoos, intimate washes, cleansers, serums, moisturizers, eye creams, balms, soaps, facial masks, sunscreens, and liquids for wet wipes.

**9.** Method for inhibiting or reducing the conversion of one or more sweat component(s) to one or more odorous compound(s), preferably on a mammal's, preferably human's, skin or mucosa, more preferably skin, most preferably on human axillary skin,
comprising or consisting of the following steps:

(a) Providing a compound of formula (I) as defined in claim 1, and
(b) bringing the compound provided in step (a) in contact with one or more microorganisms, preferably gram positive bacteria, more preferably selected from the genera *Staphylococcus, Corynebacterium,* and *Anaerococcus,* most preferably selected from the group consisting of *Staphylococcus hominis, Staphylococcus epidermidis, Corynebacterium xerosis, Corynebacterium jeikeium,* and *Anaerococcus octavius,* preferably on a mammal's, preferably human's, skin or mucosa, more preferably skin, most preferably on human axillary skin.

**10.** Compound of formula (I) as defined in claim 1 for use in the treatment of inflammation, preferably skin inflammation, post-inflammatory hyperpigmentation, atopic dermatitis, bacterial vaginosis, tinea pedis, dermatitis exfoliativa neonatorum, impetigo contagiosa, furuncles and carbuncles, folliculitis, impetigo, and/or erysipelas
and/or for use as an antimicrobial agent, preferably for inhibiting or preventing the growth of one or more micro-organisms on a mammal's, preferably human's, skin or mucosa, preferably skin, more preferably human axillary skin.

**11.** Mixture, preferably personal care, household, or pharmaceutical product, comprising one or both compound(s) of formula (I) as defined in claim 1,
preferably comprising a total amount of compound(s) of formula (I) as defined in claim 1 of from 0.01 to 10 wt.%, preferably of 0.05 to 2.5 wt.%, more preferably of 0.1 to 1 wt.%, based on the total weight of the mixture or product.

**12.** Mixture according to claim 11, comprising or consisting of

(i) one or both compound(s) of formula (I) as defined in claim 1, and
(ii) one or more further antimicrobial agent(s),
preferably wherein the total amounts of constituents (i) and (ii), respectively, contained in the mixture are such that constituent (i) synergistically improves the antimicrobial effect of constituent (ii) and/or that constituent (ii) synergistically improve(s) the antimicrobial effect of constituent (i),
more preferably wherein the one or more further antimicrobial agent(s) of constituent (ii) is/are
(ii-1) selected from the group consisting of 1,2-decanediol (Decylene Glycol), 1,2-heptanediol, 1,2-hexanediol, 1,2-nonanediol, 1,2-octanediol, 1,2-pentanediol, 1,2-propanediol, 1,3-propanediol, 2,3-decanediol, 2,3-heptanediol, 2,3-hexanediol, 2,3-nonanediol, 2,3-octanediol, 2,3-pentanediol, 2-benzylheptanol, 2-hydroxyacetophenone, 2-methyl 5-cyclohexylpentanol, 3-hydroxypropyl 2-hydroxybenzoate, 4-hydroxyacetophenone, alkyl (C 12-22) trimethyl ammonium bromide and chloride, anisic acid 3-hydroxypropylester, anisic acid and its salts, ascorbic acid and salts thereof, ascorbyl palmitate , azelaic acid, azeloyl diglycinate, benzalkonium bromide, benzalkonium chloride, benzalkonium saccharinate, benzethonium chloride, benzoic acid, benzoic acid 3-hyroxypropylester, benzyl alcohol, benzyl salicylate, benzysothiazolinone, bisabolol, bronopol (2-bromo-2-nitropropane-1,3-diol), butylene glycol, butylparaben, camphor, caprylhydroxamic acid, capryloyl glycine, capryloyl/caproyl anhydro methyl glucamide, caprylyl glyceryl ether, caprylyl/capric triglyceride, cetylpyridinium chloride, chlorhexidine, chlorhexidine diacetate, chlorhexidine digluconate, chlorhexidine dihydrochloride, chlorocresol, chloroxylenol, chlorphenesin, citronellyl methylcrotonate, climbazole, cyclohexylglycerin, dehydroacetic acid, diazolidinyl urea, dimethyl phenylbutanol, dimethyl phenylpropanol, dipropylene glycol, disodium EDTA, DMDM hydantoin, ethanol, ethyl lauroyl arginate HCl, ethyl lauroyl arginate laurate, ethylhexylglycerin, ethylparaben, eucalyptol, farnesol, garcinia mangostana peel extract, gluconic acid and salts thereof, glycerine, glyceryl caprate, glyceryl caprylate, glyceryl heptanoate, glyceryl laurate, glyceryl undecylenate, glycolipids, heptylglycerin, hexamidine, hexamidine diisethionate, hexylene glycol, hexylglycerin, hydroxyethoxyphenyl

butanol, hydroxyethoxyphenyl butanone, imidazolidinyl urea, iodopropynyl butylcarbamate, isoamyl laurate, isobutylparaben, isopropylparaben, itaconic acid and salts thereof, jasmol, lactic acid, lauryl alcohol, levulinic acid, mandelic acid, mannitol, menthol, methyl salicylate, methyl undecylenate, methylbenzyl alcohol, methylchloroisothiazolinone, methylheptylglycerin, methylisothiazolinone, methylparaben and salts thereof, methylpropanediol, morinda citrifolia callus culture lysate, octenidine HCl, octylisothiazolinone, o-cymen-5-ol , panthenol, PCA ethyl cocoyl arginate, phenethyl alcohol, phenoxyethanol, phenylpropanol, piroctone olamine, polyarginine, polyglyceryl-10 laurate, polyglyceryl-2 caprate, polyglyceryl-3 caprylate, polyglyceryl-4 caprylate/caprate, polyglyceryl-4 laurate/sebacate, polylysine, potassium sorbate, propanediol caprylate, propanediol undecylenate, propylene glycol, propylene glycol caprylate, propylparaben, raspberry ketone, saccharide isomerate, salicylic acid, salvia officinalis (sage) oil, silver chloride, silver citrate, sodium anisate, sodium benzoate, sodium caproyl lactylate, sodium caproyl/lauroyl lactylate, sodium ethylparaben, sodium lactate, sodium lauroyl lactylate, sodium levulinate, levulinic acid, sodium methylparaben, sodium phytate, phytic acid, sodium propylparaben, sorbic acid, sorbitan caprylate, sorbitol, TEA-salicylate, tetraselmis extract, tetrasodium glutamate diacetate, thymol, triclocarban, triclosan, triethyl citrate, trisodium dicarboxymethyl alaninate, trisodium ethylenediamine disuccinate, tropolone, undecylenic acid, undecylenoyl glycine, xylityl caprylate, xylityl sesquicaprylate, zinc citrate, zinc lactate, zinc neodecanoate, zinc pyrithione, zinc ricinoleate, zinc undecylenate and capryloyl/caproyl anhydro methyl glucamide (and) water.

**13.** Deodorant comprising one or both compound(s) of formula (I) as defined in claim 1.

**14.** Rinse-off product comprising one or both compound(s) of formula (I) as defined in claim 1.

**15.** Leave-on product comprising one or both compound(s) of formula (I) as defined in claim 1.

## Patentansprüche

**1.** Verwendung einer Verbindung der Formel (I)

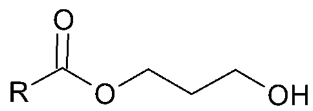

(I)

,

wobei R aus der Gruppe bestehend aus n-Nonyl und n-Undecyl ausgewählt ist,
als antimikrobielles Mittel

(i) zur Hemmung oder Verhinderung des Wachstums eines oder mehrerer Mikroorganismen in einem Körperpflege-, Haushalts- oder pharmazeutischen Produkt und/oder
(ii) zur Hemmung oder Verhinderung des Wachstums eines oder mehrerer Mikroorganismen auf einer Oberfläche.

**2.** Nicht-therapeutische Verwendung einer Verbindung der Formel (I) wie in Anspruch 1 definiert als antimikrobielles Mittel zur Hemmung oder Verhinderung des Wachstums eines oder mehrerer Mikroorganismen auf der Haut oder Schleimhaut eines Säugetiers, vorzugsweise eines Menschen, vorzugsweise auf der Haut, noch mehr bevorzugt auf der Haut einer Achselhöhle eines Menschen,
am meisten bevorzugt Verwendung einer Verbindung der Formel (I) wie in Anspruch 1 definiert als Deodorant-Wirkstoff.

**3.** Verwendung nach Anspruch 1 oder nicht-therapeutische Verwendung nach Anspruch 2, wobei der eine oder die mehreren Mikroorganismen ausgewählt ist/sind aus der Gruppe bestehend aus *Epidermophyton floccosum, Trichophyton rubrum, Staphylococcus epidermidis, Staphylococcus haemolyticus, Staphylococcus hominis, Micrococcus luteus, Acinetobacter haemolyticus, Aspergillus fumigatus, Streptococcus mutans, Porphyromonas gingivalis, Fusobacterium nucleatum, Prevotella intermedia, Malassezia furfur, Malassezia globosa, Malassezia restricta, Malassezia sympodialis, Staphylococcus capitis, Gardnerella vaginalis, Streptococcus agalactiae, Fannyhessea vaginae, Peptoniphilus lacrimalis, Cutibacterium acnes, Staphylococcus aureus, Candida albicans, Aspergillus*

*brasiliensis, Pseudomonas aeruginosa, Escherichia coli, Salmonella enteritidis, Corynebacterium xerosis, Corynebacterium jeikeium, Anaerococcus octavius, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Streptococcus pyogenes, Streptococcus pneumonia, Moraxella osloensis, Kokuria oxytoca, Kokuria pneumonia, Kokuria palustris, Kokuria rhizophilia, Sphingobium yanoikuyae, Stenotrophomonas maltophilia, Pseudomonas cremoricolorata, Bacillus subtilis, Enterobacter gergoviae, Trichophyton mentagrophytes, Brevibacterium epidermidis, Klebsiella pneumonia, Pseudomonas fluorescens, Pseudomonas putida, Penicillium funiculosum, Bacillus licheniformis* und *Enterococcus hirae.*

**4.** Verwendung nach Anspruch 1 oder 3,

wobei das Körperpflegeprodukt ausgewählt ist aus der Gruppe bestehend aus Deodorantprodukten, Körperpflege- und Körperreinigungsprodukten, Intimpflege- und Intimreinigungsprodukten, Fußpflege- und Fußreinigungsprodukten, Mundpflege- und Mundreinigungsprodukten, Kopfhaut- und Haarpflegeprodukten, Kopfhaut- und Haarreinigungsprodukten und Gesichtspflege- und Gesichtsreinigungsprodukten, wobei das Körperpflegeprodukt vorzugsweise ausgewählt ist aus der Gruppe bestehend aus Deodorantsprays, Deodorant-Aerosolen, Deodorant-Roll-ons, Deodorant-Sticks, Deodorant-Cremes, Fußcremes, Fußpudern, Körperwaschmitteln, Mundwässern, Zahnpasten, Shampoos, Haarspülungen, Stylingwachsen, Stylinggelen, Stylingschäumen, Stylingcremes, Stylingmousses, Stylingseren, Stylingpomaden, Mizellenwässern, Intimwaschmitteln, Reinigungsmitteln, Tonern, Seren, Feuchtigkeitscremes, Augencremes, Balsamen, Seifen, Gesichtsmasken, Sonnenschutzmitteln, Flüssigkeiten für Feuchttücher, Grundierungen, Concealer, Foundations, Rouges, Bronzer, Highlighter, Augenbrauenstifte, Augenbrauencremes, Augenbrauenwachse, Augenbrauengele, Augenbrauenpuder, Lidschatten, Eyeliner, Mascara, Lippenstifte, Lipgloss, Lipliner, Lippenbalsam, Gesichtspuder, Fixierpuder, Fixiersprays und Nagellacke,
und/oder
wobei das Haushaltsprodukt ausgewählt ist aus der Gruppe bestehend aus alkoholfreien Glasreinigern, Glasreinigern, Allzweckreinigern, Allzwecksprays, Entfettungsmitteln, Teppichreinigern, Holzreinigern, Bodenreinigern, Laminatreinigern, Vinylreinigern, Keramikreinigern, Korkreinigern, Linoleumreinigern, Porzellanreinigern, Steinreinigern, Betonreinigern, Parkettreinigern, Hochglanz-Bodenreinigern, Küchenreinigern, Fettlösern, Badreinigern, Toilettenreinigern, Kalkreinigern, Geschirrspülmittel für Geschirrspülmaschinen, Handspülmitteln, antibakterielle Handspülmitteln, Weichspülern, Textilpflegeprodukten, Flüssigwaschmitteln, Waschpulvern und Hygienewaschmitteln,
und/oder
wobei das pharmazeutische Produkt ausgewählt ist aus der Gruppe bestehend aus Deodorant-Sprays, Deodorant-Roll-ons, Deodorant-Sticks, Deodorant-Cremes, Fußcremes, Körperwaschmitteln, Mundspülungen, Zahnpasten, Shampoos, Intimwaschmitteln, Reinigungsmitteln, Seren, Feuchtigkeitscremes, Augencremes, Balsamen, Seifen, Gesichtsmasken, Sonnenschutzmitteln und Flüssigkeiten für Feuchttücher.

**5.** Verwendung nach Anspruch 1 oder 3, wobei die Oberfläche eine feste Oberfläche ist, vorzugsweise ausgewählt aus der Gruppe bestehend aus Küchenoberflächen, Badezimmeroberflächen, Waschmaschinentrommeln, Besteck, Geschirr, Holzfußböden (Massivholzböden, Fertigparkett), Laminat, Vinyl, Keramik, Kork, Linoleum, Porzellan, Stein, Beton und Parkett, oder eine nicht feste Oberfläche ist, vorzugsweise ausgewählt aus der Gruppe bestehend aus gewebten Materialien und Ledern, noch mehr bevorzugt Stoffen, Teppichen, Tüchern, Kleidungsstücken, Laken und Schwämmen.

**6.** Verfahren

(i) zur Hemmung oder Verhinderung des Wachstums eines oder mehrerer Mikroorganismen in einem Körperpflege-, Haushalts- oder pharmazeutischen Produkt und/oder
(ii) zur Hemmung oder Verhinderung des Wachstums eines oder mehrerer Mikroorganismen auf/in einer Oberfläche,

umfassend oder bestehend aus den folgenden Schritten:

(a) Bereitstellen einer Verbindung der Formel (I) wie in Anspruch 1 definiert und
(b) Inkontaktbringen der in Schritt (a) bereitgestellten Verbindung

(i) mit den anderen Inhaltsstoffen eines Körperpflege-, Haushalts- oder pharmazeutischen Produkts, in dem

das Wachstum eines oder mehrerer Mikroorganismen gehemmt oder verhindert werden soll, und/oder (ii) mit einer Oberfläche, auf/in der das Wachstum eines oder mehrerer Mikroorganismen gehemmt oder verhindert werden soll.

**7.** Verfahren nach Anspruch 6, wobei der eine oder die mehreren Mikroorganismen, dessen/deren Wachstum gehemmt oder verhindert wird, ausgewählt ist/sind aus der Gruppe bestehend aus *Epidermophyton floccosum, Trichophyton rubrum, Staphylococcus epidermidis, Staphylococcus haemolyticus, Staphylococcus hominis, Micrococcus luteus, Acinetobacter haemolyticus, Aspergillus fumigatus, Streptococcus mutans, Porphyromonas gingivalis, Fusobacterium nucleatum, Prevotella intermedia, Malassezia furfur, Malassezia globosa, Malassezia restricta, Malassezia sympodialis, Staphylococcus capitis, Gardnerella vaginalis, Streptococcus agalactiae, Fannyhessea vaginae, Peptoniphilus lacrimalis, Cutibacterium acnes, Staphylococcus aureus, Candida albicans, Aspergillus brasiliensis, Pseudomonas aeruginosa, Escherichia coli, Salmonella enteritidis, Corynebacterium xerosis, Corynebacterium jeikeium, Anaerococcus octavius, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Streptococcus pyogenes, Streptococcus pneumonia, Moraxella osloensis, Kokuria oxytoca, Kokuria pneumonia, Kokuria palustris, Kokuria rhizophilia, Sphingobium yanoikuyae, Stenotrophomonas maltophilia, Pseudomonas cremoricolorata, Bacillus subtilis, Enterobacter gergoviae, Trichophyton mentagrophytes, Brevibacterium epidermidis, Klebsiella pneumonia, Pseudomonas fluorescens, Pseudomonas putida, Penicillium funiculosum, Bacillus licheniformis* und *Enterococcus hirae.*

**8.** Verfahren nach Anspruch 6 oder 7,

wobei das Körperpflegeprodukt ausgewählt ist aus der Gruppe bestehend aus Deodorantprodukten, Körperpflege- und Körperreinigungsprodukten, Intimpflege- und Intimreinigungsprodukten, Fußpflege- und Fußreinigungsprodukten, Mundpflege- und Mundreinigungsprodukten, Kopfhautpflege und Kopfhautreinigungsprodukten und Gesichtspflege- und Gesichtsreinigungsprodukten, wobei das Körperpflegeprodukt vorzugsweise ausgewählt ist aus der Gruppe bestehend aus Deodorantsprays, Deodorant-Roll-ons, Deodorant-Sticks, Deodorant-Cremes, Fußcremes, Körperwaschmitteln, Mundwässern, Zahnpasten, Shampoos, Haarspülungen, Stylingwachsen, Stylinggelen, Stylingschäumen, Stylingcremes, Stylingmousses, Stylingseren, Stylingpomaden, Mizellenwässern, Intimwaschmitteln, Reinigungsmitteln, Tonern, Seren, Feuchtigkeitscremes, Augencremes, Balsamen, Seifen, Gesichtsmasken, Sonnenschutzmitteln, Flüssigkeiten für Feuchttücher, Grundierungen, Concealer, Foundations, Rouges, Bronzer, Highlighter, Augenbrauenstifte, Augenbrauencremes, Augenbrauenwachse, Augenbrauengele, Augenbrauenpuder, Lidschatten, Eyeliner, Mascara, Lippenstifte, Lipgloss, Lipliner, Lippenbalsam, Gesichtspuder, Fixierpuder, Fixiersprays und Nagellacke,
und/oder
wobei das Haushaltsprodukt ausgewählt ist aus der Gruppe bestehend aus alkoholfreien Glasreinigern, Glasreinigern, Allzweckreinigern, Allzwecksprays, Entfettungsmitteln, Teppichreinigern, Holzreinigern, Bodenreinigern, Laminatreinigern, Vinylreinigern, Keramikreinigern, Korkreinigern, Linoleumreinigern, Porzellanreinigern, Steinreinigern, Betonreinigern, Parkettreinigern, Hochglanz-Bodenreinigern, Küchenreinigern, Fettlösern, Badreinigern, Toilettenreinigern, Kalkreinigern, Geschirrspülmittel für Geschirrspülmaschinen, Handspülmitteln, antibakterielle Handspülmitteln, Weichspülern, Textilpflegeprodukten, Flüssigwaschmitteln, Waschpulvern und Hygienewaschmitteln,
und/oder
wobei das pharmazeutische Produkt ausgewählt ist aus der Gruppe bestehend aus Deodorant-Sprays, Deodorant-Roll-ons, Deodorant-Sticks, Deodorant-Cremes,
Fußcremes, Körperwaschmitteln, Mundspülungen, Zahnpasten, Shampoos, Intimwaschmitteln, Reinigungsmitteln, Seren, Feuchtigkeitscremes, Augencremes, Balsamen, Seifen, Gesichtsmasken, Sonnenschutzmitteln und Flüssigkeiten für Feuchttücher.

**9.** Verfahren zur Hemmung oder Reduzierung der Umwandlung einer oder mehrerer Schweißkomponente(n) in eine oder mehrere geruchsintensive Verbindung(en), vorzugsweise auf der Haut oder Schleimhaut eines Säugetiers, vorzugsweise eines Menschen, noch mehr bevorzugt auf der Haut, am meisten bevorzugt auf der Haut einer Achselhöhle eines Menschen,
umfassend oder bestehend aus den folgenden Schritten:

(a) Bereitstellen einer Verbindung der Formel (I) nach Anspruch 1 und
(b) Inkontaktbringen der in Schritt (a) bereitgestellten Verbindung mit einem oder mehreren Mikroorganismen, vorzugsweise grampositiven Bakterien, noch mehr bevorzugt ausgewählt aus den Gattungen *Staphylococcus,*

*Corynebacterium* und *Anaerococcus,* am meisten bevorzugt ausgewählt aus der Gruppe bestehend aus *Staphylococcus hominis, Staphylococcus epidermidis, Corynebacterium xerosis, Corynebacterium jeikeium* und *Anaerococcus octavius,* vorzugsweise auf der Haut oder Schleimhaut eines Säugetiers, vorzugsweise eines Menschen, noch mehr bevorzugt auf der Haut, am meisten bevorzugt auf der Haut einer Achselhöhle eines Menschen.

**10.** Verbindung der Formel (I) wie in Anspruch 1 definiert zur Verwendung bei der Behandlung von Entzündungen, vorzugsweise Hautentzündungen, postinflammatorischer Hyperpigmentierung, atopischer Dermatitis, bakterieller Vaginose, Tinea pedis, Dermatitis exfoliativa neonatorum, Impetigo contagiosa, Furunkeln und Karbunkeln, Follikulitis, Impetigo und/oder Wundrose
und/oder zur Verwendung als antimikrobielles Mittel, vorzugsweise zur Hemmung oder Verhinderung des Wachstums eines oder mehrerer Mikroorganismen auf der Haut oder Schleimhaut eines Säugetiers, vorzugsweise eines Menschen, vorzugsweise auf der Haut, noch mehr bevorzugt auf der Haut einer Achselhöhle eines Menschen.

**11.** Mischung, vorzugsweise Körperpflege-, Haushalts- oder pharmazeutisches Produkt, umfassend eine oder beide Verbindungen der Formel (I) wie in Anspruch 1 definiert,
vorzugsweise umfassend einen Gesamtgehalt an Verbindung(en) der Formel (I) wie in Anspruch 1 definiert von 0,01 bis 10 Gew.-%, vorzugsweise von 0,05 bis 2,5 Gew.-%, noch mehr bevorzugt von 0,1 bis 1 Gew.-%, bezogen auf das Gesamtgewicht der Mischung oder des Produkts.

**12.** Mischung nach Anspruch 11, umfassend oder bestehend aus

(i) einer oder beiden Verbindungen der Formel (I) wie in Anspruch 1 definiert und
(ii) einem oder mehreren weiteren antimikrobiellen Wirkstoffen,
vorzugsweise wobei die Gesamtmengen der in der Mischung enthaltenen Bestandteile (i) und (ii) jeweils so bemessen sind, dass der Bestandteil (i) die antimikrobielle Wirkung des Bestandteils (ii) synergistisch verbessert und/oder dass der Bestandteil (ii) die antimikrobielle Wirkung des Bestandteils (i) synergistisch verbessert,
vorzugsweise wobei der eine oder die mehreren weiteren antimikrobiellen Wirkstoffe des Bestandteils (ii)
(ii-1) ausgewählt ist/sind aus der Gruppe bestehend aus 1,2-Decandiol (Decylenglykol), 1,2-Heptandiol, 1,2-Hexandiol, 1,2-Nonandiol, 1,2-Octandiol, 1,2-Pentandiol, 1,2-Propandiol, 1,3-Propandiol, 2,3-Decandiol, 2,3-Heptandiol, 2,3-Hexandiol, 2,3-Nonandiol, 2,3-Octandiol, 2,3-Pentandiol, 2-Benzylheptanol, 2-Hydroxyacetophenon, 2-Methyl-5-cyclohexylpentanol, 3-Hydroxypropyl-2-hydroxybenzoat, 4-Hydroxyacetophenon, Alkyl (C 12-22) trimethylammoniumbromid und -chlorid, Anisinsäure-3-hydroxypropylester, Anissäure und ihre Salze, Ascorbinsäure und ihre Salze, Ascorbylpalmitat, Azelainsäure, Azeloyldiglycinat, Benzalkoniumbromid, Benzalkoniumchlorid, Benzalkoniumsaccharinat, Benzethoniumchlorid, Benzoesäure, Benzoesäure-3-hydroxypropylester, Benzylalkohol, Benzylsalicylat, Benzisothiazolinon, Bisabolol, Bronopol (2-Brom-2-nitropropan-1,3-diol), Butylenglykol, Butylparaben, Kampfer, Caprylhydroxamsäure, Capryloylglycin, Capryloyl/Caproylanhydro-methylglucamid, Caprylylglycerylether, Caprylyl-/Capric-Triglycerid, Cetylpyridiniumchlorid, Chlorhexidin, Chlorhexidindiacetat, Chlorhexidindigluconat, Chlorhexidindihydrochlorid, Chlorocresol, Chloroxylenol, Chlorphenesin, Citronellylmethylcrotonat, Climbazol, Cyclohexylglycerin, Dehydroessigsäure, Diazolidinylharnstoff, Dimethylphenylbutanol, Dimethylphenylpropanol, Dipropylenglykol, Dinatrium-EDTA, DMDM-Hydantoin, Ethanol, Ethyllauroylarginat-HCl, Ethyllauroylarginatlaurat, Ethylhexylglycerin, Ethylparaben, Eukalyptol, Farnesol, Garcinia-Mangostana-Schalenextrakt, Gluconsäure und deren Salze, Glycerin, Glycerylcaprat, Glycerylcaprylat, Glycerylheptanoat, Glyceryllaurat, Glycerylundecylenat, Glykolipide, Heptylglycerin, Hexamidin, Hexamidindiisethionat, Hexylenglykol, Hexylglycerin, Hydroxyethoxyphenylbutanol, Hydroxyethoxyphenylbutanon, Imidazolidinylharnstoff, Iodopropynylbutylcarbamat, Isoamyllaurat, Isobutylparaben, Isopropylparaben, Itaconsäure und deren Salze, Jasmol, Milchsäure, Laurylalkohol, Levulinsäure, Mandelsäure, Mannitol, Menthol, Methylsalicylat, Methylundecylenat, Methylbenzylalkohol, Methylchloroisothiazolinon, Methylheptylglycerin, Methylisothiazolinon, Methylparaben und Salze davon, Methylpropandiol, Morinda citrifolia-Kalluskulturlysat, Octenidin-HCl, Octylisothiazolinon, o-Cymen-5-ol, Panthenol, PCA-Ethylcocoylarginat, Phenethylalkohol, Phenoxyethanol, Phenylpropanol, Piroctonolamin, Polyarginin, Polyglyceryl-10-Laurat, Polyglyceryl-2-Caprat, Polyglyceryl-3-Caprylat, Polyglyceryl-4-Caprylat/Caprat, Polyglyceryl-4-Laurat/Sebacat, Polylysin, Kaliumsorbat, Propandiolcaprylat, Propandiolundecylenat, Propylenglykol, Propylenglycolcaprylat, Propylparaben, Himbeerketon, Saccharidisomerat, Salicylsäure, Salvia officinalis (Salbei)-Öl, Silberchlorid, Silbercitrat, Natriumanisat, Natriumbenzoat, Natriumcaproyl-Lactylat, Natriumcaproyl-/Lauroyl-Lactylat, Natriumethylparaben, Natriumlactat, Natriumlauroyl-Lactylat, Natriumlevulinat, Levulinsäure, Natriummethylparaben, Natriumphytat, Phytinsäure, Natriumpropylparaben, Sorbinsäure, Sorbitancaprylat, Sorbitol, TEA-Salicylat, Tetraselmis-Extrakt, Tetranatriumglutamatdiacetat, Thymol, Triclocarban, Triclosan, Triethylcitrat, Trinatriumdicarboxymethylalaninat,

Trinatriumethylendiamindisuccinat, Tropolon, Undecylensäure, Undecylenoylglycin, Xylitylcaprylat, Xylitylsesquicaprylat, Zinkcitrat, Zinklactat, Zinkneodecanoat, Zinkpyrithion, Zinkricinoleat, Zinkundecylenat und Caproyl/Caproylanhydro-Methylglucamid (und) Wasser.

**13.** Deodorant umfassend eine oder beide Verbindungen der Formel (I) wie in Anspruch 1 definiert.

**14.** Abwaschbares Produkt umfassend eine oder beide Verbindungen der Formel (I) wie in Anspruch 1 definiert.

**15.** Leave-on-Produkt umfassend eine oder beide Verbindungen der Formel (I) wie in Anspruch 1 definiert.

## Revendications

**1.** Utilisation d'un composé de formule (I)

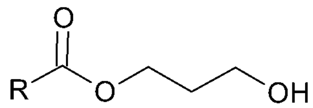

(I)

,

dans laquelle R est choisi dans le groupe constitué du n-nonyle et du n-undécyle,
comme agent antimicrobien

(i) pour inhiber ou prévenir la croissance d'un ou de plusieurs micro-organismes dans un produit de soin personnel, un produit ménager ou un produit pharmaceutique, et/ou
(ii) pour inhiber ou prévenir la croissance d'un ou de plusieurs micro-organismes sur une surface.

**2.** Utilisation non thérapeutique d'un composé de formule (I) tel que défini dans la revendication 1, comme agent antimicrobien pour inhiber ou prévenir la croissance d'un ou de plusieurs micro-organismes sur la peau ou la muqueuse d'un mammifère, de préférence d'un être humain, de préférence sur la peau, de manière plus préférée sur la peau axillaire humaine,
de manière la plus préférée, l'utilisation d'un composé de formule (I) tel que défini dans la revendication 1 comme principe actif de déodorant.

**3.** Utilisation selon la revendication 1 ou utilisation non-thérapeutique selon la revendication 2, dans laquelle ledit un ou lesdits plusieurs micro-organismes est/sont choisis dans le groupe constitué de *Epidermophyton floccosum, Trichophyton rubrum, Staphylococcus epidermidis, Staphylococcus haemolyticus, Staphylococcus hominis, Micrococcus luteus, Acinetobacter haemolyticus, Aspergillus fumigatus, Streptococcus mutans, Porphyromonas gingivalis, Fusobacterium nucleatum, Prevotella intermedia, Malassezia furfur, Malassezia globosa, Malassezia restricta, Malassezia sympodialis, Staphylococcus capitis, Gardnerella vaginalis, Streptococcus agalactiae, Fannyhessea vaginae, Peptoniphilus lacrimalis, Cutibacterium acnes, Staphylococcus aureus, Candida albicans, Aspergillus brasiliensis, Pseudomonas aeruginosa, Escherichia coli, Salmonella enteritidis, Corynebacterium xerosis, Corynebacterium jeikeium, Anaerococcus octavius, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Streptococcus pyogenes, Streptococcus pneumonia, Moraxella osloensis, Kokuria oxytoca, Kokuria pneumonia, Kokuria palustris, Kokuria rhizophilia, Sphingobium yanoikuyae, Stenotrophomonas maltophilia, Pseudomonas cremoricolorata, Bacillus subtilis, Enterobacter gergoviae, Trichophyton mentagrophytes, Brevibacterium epidermidis, Klebsiella pneumonia, Pseudomonas fluorescens, Pseudomonas putida, Penicillium funiculosum, Bacillus licheniformis,* et *Enterococcus hirae.*

**4.** Utilisation selon la revendication 1 ou 3,

dans laquelle le produit de soin personnel est choisi dans le groupe constitué par les produits de déodorant, les produits de soin et de nettoyage du corps, les produits de soin et de nettoyage intime, les produits de soin et de nettoyage des pieds, les produits de soin et de nettoyage bucco-dentaire, les produits de soin et de nettoyage du cuir chevelu et des cheveux, et les produits de soin et de nettoyage du visage, plus préférablement dans laquelle

le produit de soin personnel est choisi dans le groupe constitué par les déodorants en spray, les déodorants en aérosol, les déodorants roll-on, les déodorants en stick, les crèmes déodorantes, les crèmes pour les pieds, les poudres pour les pieds, les produits de lavage du corps, les bains de bouche, les dentifrices, les shampoings, les après-shampoings, les cires coiffantes, les gels coiffants, les mousses coiffantes, les crèmes coiffantes, les mousses capillaires, les sérums coiffants, les pommades coiffantes, les eaux micellaires, les produits de lavage intime, les nettoyants, les toniques, les sérums, les crèmes hydratantes, les crèmes pour les yeux, les baumes, les savons, les masques faciaux, les écrans solaires, les liquides pour lingettes humides, les bases de teint, les correcteurs, les fonds de teint, les fards à joues, les poudres bronzantes, les illuminateurs, les crayons à sourcils, les crèmes pour les sourcils, les cires à sourcils, les gels à sourcils, les poudres à sourcils, les ombres à paupières, les eye-liners, les mascaras, les rouges à lèvres, les gloss, les crayons à lèvres, les baumes à lèvres, les poudres pour le visage, les poudres fixatrices, les sprays fixateurs et les vernis à ongles
et/ou
dans laquelle le produit ménager est choisi dans le groupe constitué par les nettoyants pour vitres sans alcool, les nettoyants pour vitres, les nettoyants multi-usages, les sprays multi-usages, les dégraissants, les nettoyants pour tapis, les nettoyants pour bois, les nettoyants pour sols, les nettoyants pour stratifiés, les nettoyants pour vinyle, les nettoyants pour céramique, les nettoyants pour liège, les nettoyants pour linoléum, les nettoyants pour porcelaine, les nettoyants pour pierre, les nettoyants pour béton, les nettoyants pour parquet, les nettoyants pour sols brillants, les nettoyants pour cuisine, les nettoyants dégraissants, les nettoyants pour salle de bain, les nettoyants pour toilettes, les nettoyants pour calcaire, les liquides vaisselles pour lave-vaisselle, les liquides vaisselles, les liquides vaisselles antibactériens, les adoucissants, les produits d'entretien du linge, les lessives liquides, les lessives en poudre et les lessives d'hygiène,
et/ou
dans laquelle le produit pharmaceutique est choisi dans le groupe constitué par les déodorants en spray, les déodorants roll-on, les déodorants en stick, les crèmes déodorantes, les crèmes pour les pieds, les produits de nettoyage du corps, les bains de bouche, les dentifrices, les shampoings, les produits d'hygiène intime, les nettoyants, les sérums, les crèmes hydratantes, les crèmes pour les yeux, les baumes, les savons, les masques faciaux, les écrans solaires et les liquides pour lingettes humides.

**5.** Utilisation selon la revendication 1 ou 3, dans laquelle la surface est une surface solide, de préférence choisie dans le groupe constitué par les surfaces de cuisine, les surfaces de salle de bain, les tambours de machines à laver, les couverts, la vaisselle, les revêtements de sol en bois (revêtements de sol massif, revêtements de sol contrecollé), les stratifiés, le vinyle, la céramique, le liège, le linoléum, la porcelaine, la pierre, le béton et le parquet, ou est une surface non solide, de préférence choisie dans le groupe constitué par les matières tissées et les cuirs, de manière plus préférée les tissus, les tapis, les toiles, les vêtements, les draps et les éponges.

**6.** Procédé

(i) pour inhiber ou prévenir la croissance d'un ou de plusieurs micro-organismes dans un produit de soin personnel, un produit ménager ou un produit pharmaceutique, et/ou
(ii) pour inhiber ou prévenir la croissance d'un ou de plusieurs micro-organismes sur/dans une surface,

comprenant ou consistant en les étapes suivantes:

(a) fournir un composé de formule (I) tel que défini dans la revendication 1, et
(b) mettre le composé fourni à l'étape (a)

(i) en contact avec les autres ingrédients d'un produit de soin personnel, d'un produit ménager ou d'un produit pharmaceutique, dans lequel la croissance d'un ou de plusieurs micro-organismes doit être inhibée ou empêchée, et/ou
(ii) en contact avec une surface sur/dans laquelle la croissance d'un ou de plusieurs micro-organismes doit être inhibée ou empêchée.

**7.** Pocédé selon la revendication 6, dans lequel ledit un ou lesdits plusieurs micro-organismes dont la croissance est inhibée ou empêchée est/sont choisi(s) dans le groupe constitué par *Epidermophyton floccosum, Trichophyton rubrum, Staphylococcus epidermidis, Staphylococcus haemolyticus, Staphylococcus hominis, Micrococcus luteus, Acinetobacter haemolyticus, Aspergillus fumigatus, Streptococcus mutans, Porphyromonas gingivalis, Fusobacterium nucleatum, Prevotella intermedia, Malassezia furfur, Malassezia globosa, Malassezia restricta, Malassezia sympodialis, Staphylococcus capitis, Gardnerella vaginalis, Streptococcus agalactiae, Fannyhessea vaginae,*

*Peptoniphilus lacrimalis, Cutibacterium acnes, Staphylococcus aureus, Candida albicans, Aspergillus brasiliensis, Pseudomonas aeruginosa, Escherichia coli, Salmonella enteritidis, Corynebacterium xerosis, Corynebacterium jeikeium, Anaerococcus octavius, Acinetobacter ursingii, Acinetobacter pittii, Acinetobacter johnsonii, Agrobacterium tumefaciens, Brevundimonas diminuta, Campylobacter jejuni, Campylobacter coli, Chryseobacterium hominis, Chryseobacterium haifense, Clostridium perfringens, Streptococcus pyogenes, Streptococcus pneumonia, Moraxella osloensis, Kokuria oxytoca, Kokuria pneumonia, Kokuria palustris, Kokuria rhizophilia, Sphingobium yanoikuyae, Stenotrophomonas maltophilia, Pseudomonas cremoricolorata, Bacillus subtilis, Enterobacter gergoviae, Trichophyton mentagrophytes, Brevibacterium epidermidis, Klebsiella pneumonia, Pseudomonas fluorescens, Pseudomonas putida, Penicillium funiculosum, Bacillus licheniformis,* et *Enterococcus hirae.*

**8.** Procédé selon la revendication 6 ou 7,

dans lequel le produit de soin personnel est choisi dans le groupe constitué par les produits de déodorant, les produits de soin et de nettoyage du corps, les produits de soin et de nettoyage intime, les produits de soin et de nettoyage des pieds, les produits de soin et de nettoyage bucco-dentaire, les produits de soin et de nettoyage du cuir chevelu et des cheveux, et les produits de soin et de nettoyage du visage, plus préférablement dans lequel le produit de soin personnel est choisi dans le groupe constitué par les déodorants en spray, les déodorants roll-on, les déodorants en stick, les crèmes déodorantes, les crèmes pour les pieds, les produits de lavage du corps, les bains de bouche, les dentifrices, les shampoings, les après-shampoings, les cires coiffantes, les gels coiffants, les mousses coiffantes, les crèmes coiffantes, les mousses capillaires, les sérums coiffants, les pommades coiffantes, les eaux micellaires, les produits de lavage intime, les nettoyants, les toniques, les sérums, les crèmes hydratantes, les crèmes pour les yeux, les baumes, les savons, les masques faciaux, les écrans solaires, les liquides pour lingettes humides, les bases de teint, les correcteurs, les fonds de teint, les fards à joues, les poudres bronzantes, les illuminateurs, les crayons à sourcils, les crèmes pour les sourcils, les cires à sourcils, les gels à sourcils, les poudres à sourcils, les ombres à paupières, les eye-liners, les mascaras, les rouges à lèvres, les gloss, les crayons à lèvres, les baumes à lèvres, les poudres pour le visage, les poudres fixatrices, les sprays fixateurs et les vernis à ongles
et/ou
dans lequel le produit ménager est choisi dans le groupe constitué par les nettoyants pour vitres sans alcool, les nettoyants pour vitres, les nettoyants multi-usages, les sprays multi-usages, les dégraissants, les nettoyants pour tapis, les nettoyants pour bois, les nettoyants pour sols, les nettoyants pour stratifiés, les nettoyants pour vinyle, les nettoyants pour céramique, les nettoyants pour liège, les nettoyants pour linoléum, les nettoyants pour porcelaine, les nettoyants pour pierre, les nettoyants pour béton, les nettoyants pour parquet, les nettoyants pour sols brillants, les nettoyants pour cuisine, les nettoyants dégraissants, les nettoyants pour salle de bain, les nettoyants pour toilettes, les nettoyants pour calcaire, les liquides vaisselles pour lave-vaisselle, les liquides vaisselles, les liquides vaisselles antibactériens, les adoucissants, les produits d'entretien du linge, les lessives liquides, les lessives en poudre et les lessives d'hygiène,
et/ou
dans lequel le produit pharmaceutique est choisi dans le groupe constitué par les déodorants en spray, les déodorants roll-on, les déodorants en stick, les crèmes déodorantes, les crèmes pour les pieds, les produits de lavage du corps, les bains de bouche, les dentifrices, les shampoings, les produits de lavage intime, les nettoyants, les sérums, les crèmes hydratantes, les crèmes pour les yeux, les baumes, les savons, les masques pour le visage, les écrans solaires et les liquides pour lingettes humides.

**9.** Procédé pour inhiber ou réduire la conversion d'un ou de plusieurs composant(s) de la sueur en un ou plusieurs composé(s) odorant(s), de préférence sur la peau ou la muqueuse d'un mammifère, de préférence d'un être humain, plus préférablement sur la peau, de manière la plus préférée sur la peau axillaire humaine,
comprenant ou consistant en les étapes suivantes:

(a) fournir un composé de formule (I) tel que défini dans la revendication 1, et
(b) mettre le composé fourni à l'étape (a) en contact avec un ou plusieurs micro-organismes, de préférence des bactéries Gram positif, de manière plus préférée choisies parmi les genres *Staphylococcus, Corynebacterium,* et *Anaerococcus,* de manière la plus préférée choisies dans le groupe constitué de *Staphylococcus hominis, Staphylococcus epidermidis, Corynebacterium xerosis, Corynebacterium jeikeium,* et *Anaerococcus octavius,* de préférence sur la peau ou la muqueuse d'un mammifère, de préférence d'un être humain, plus préférablement sur la peau, de manière la plus préférée sur la peau axillaire humaine.

**10.** Composé de formule (I) tel que défini dans la revendication 1, destiné à être utilisé dans le traitement de

l'inflammation, de préférence de l'inflammation cutanée, de l'hyperpigmentation post-inflammatoire, de la dermatite atopique, de la vaginose bactérienne, du tinea pedis, de la dermatite exfoliative néonatale, de l'impétigo contagieux, des furoncles et anthrax, de la folliculite, de l'impétigo et/ou de l'érysipèle
et/ou destiné à être utilisé comme agent antimicrobien, de préférence pour inhiber ou prévenir la croissance d'un ou de plusieurs micro-organismes sur la peau ou les muqueuses d'un mammifère, de préférence d'un être humain, de préférence sur la peau, de manière plus préférée sur la peau axillaire humaine.

**11.** Mélange, de préférence produit de soin personnel, produit ménager ou produit pharmaceutique, comprenant un ou les deux composé(s) de formule (I) tels que définis dans la revendication 1,
comprenant de préférence une quantité totale de composé(s) de formule (I) tel que défini dans la revendication 1, comprise entre 0,01 et 10 % en poids, de préférence entre 0,05 et 2,5 % en poids, de manière plus préférée entre 0,1 et 1 % en poids, par rapport au poids total du mélange ou du produit.

**12.** Mélange selon la revendication 11, comprenant ou constitué de

(i) un ou les deux composés de formule (I) tels que définis dans la revendication 1, et
(ii) un ou plusieurs autre(s) agent(s) actif(s) antimicrobien(s),
de préférence, les quantités totales des constituants (i) et (ii), respectivement, contenus dans le mélange étant telles que le constituant (i) améliore de façon synergique l'effet antimicrobien du constituant (ii) et/ou que le constituant (ii) améliore de façon synergique l'effet antimicrobien du constituant (i),
plus préférablement, dans lequel ledit un ou lesdits plusieurs autre(s) agent(s) actif(s) antimicrobien(s) du constituant (ii) est/sont
(ii-1) choisi(s) dans le groupe constitué par le 1,2-décanediol (décylène glycol), le 1,2-heptanediol, le 1,2-hexanediol, le 1,2-nonanediol, le 1,2-octanediol, le 1,2-pentanediol, le 1,2-propanediol, 1,3-propanediol, 2,3-décanediol, 2,3-heptanediol, 2,3-hexanediol, 2,3-nonanediol, 2,3-octanediol, 2,3-pentanediol, 2-benzylheptanol, 2-hydroxyacétophénone, 2-méthyl-5-cyclohexylpentanol, 2-hydroxybenzoate de 3-hydroxypropyle, 4-hydroxyacétophénone, bromure et chlorure d'alkyl(C12-C22)triméthylammonium, ester 3-hydroxypropylique de l'acide anisique, acide anisique et ses sels, acide ascorbique et ses sels, palmitate d'ascorbyle, acide azélaïque, diglycinate d'azéloyle, bromure de benzalkonium, chlorure de benzalkonium, saccharinate de benzalkonium, chlorure de benzéthonium, acide benzoïque, 3-hydroxypropylester de l'acide benzoïque, alcool benzylique, salicylate de benzyle, benzysothiazolinone, bisabolol, bronopol (2-bromo-2-nitropropane-1,3-diol), butylène glycol, butylparabène, camphre, acide caprylhydroxamique, capryloylglycine, capryloyl/caproylanhydrométhylglucamide, éther caprylylique de glycéryle, triglycéride caprylylique/caprique, chlorure de cétylpyridinium, chlorhexidine, diacétate de chlorhexidine, digluconate de chlorhexidine, dichlorhydrate de chlorhexidine, chlorocrésol, chloroxylénol, chlorphénésine, méthylcrotonate de citronellyle, climbazole, cyclohexylglycérine, acide déhydroacétique, diazolidinylurée, diméthylphénylbutanol, diméthylphénylpropanol, dipropylène glycol, disodium EDTA, DMDM hydantoïne, éthanol, chlorhydrate d'arginate d'éthyle lauroyl, laurate d'arginate d'éthyle lauroyl, éthylhexylglycérine, éthylparabène, eucalyptol, farnésol, extrait d'écorce de Garcinia mangostana, acide gluconique et ses sels, glycérine, caprate de glycéryle, caprylate de glycéryle, heptanoate de glycéryle, laurate de glycéryle, undécylénate de glycéryle, glycolipides, heptylglycérine, hexamidine, diiséthionate d'hexamidine, hexylène glycol, hexylglycérine, hydroxyéthoxyphényl butanol, hydroxyéthoxyphényl butanone, imidazolidinylurée, iodopropynyl butylcarbamate, laurate d'isoamyle, isobutylparabène, isopropylparabène, acide itaconique et ses sels, jasmol, acide lactique, alcool laurylique, acide lévulinique, acide mandélique, mannitol, menthol, salicylate de méthyle, undécylénate de méthyle, alcool méthylbenzylique, méthylchloroisothiazolinone, méthylheptylglycérine, méthylisothiazolinone, méthylparabène et ses sels, méthylpropanediol, lysat de culture de cals de Morinda citrifolia, chlorhydrate d'octénidine, octylisothiazolinone, o-cymène-5-ol, panthénol, PCA éthyl cocoyl arginate, alcool phénéthylique, phénoxyéthanol, phénylpropanol, piroctone olamine, polyarginine, laurate de polyglycéryl-10, caprate de polyglycéryl-2, caprylate de polyglycéryl-3, caprylate/caprate de polyglycéryl-4, laurate/sébacate de polyglycéryl-4, polylysine, sorbate de potassium, caprylate de propanediol, undécylénate de propanediol, propylène glycol, caprylate de propylène glycol, propylparabène, cétone de framboise, isomérate de saccharide, acide salicylique, huile de Salvia officinalis (sauge), chlorure d'argent, citrate d'argent, sodium anisate, benzoate de sodium, caproyl lactylate de sodium, caproyl/lauroyl lactylate de sodium, éthylparabène de sodium, lactate de sodium, lauroyl lactylate de sodium, lévulinate de sodium, acide lévulinique, méthylparabène de sodium, phytate de sodium, acide phytique, propylparabène de sodium, acide sorbique, caprylate de sorbitan, sorbitol, salicylate de TEA, extrait de Tetraselmis, diacétate de glutamate tétrasodique, thymol, triclocarban, triclosan, citrate de triéthyle, dicarboxyméthyl alaninate trisodique, disuccinate d'éthylènediamine trisodique, tropolone, acide undécylénique, undécylénoyl glycine, caprylate de xylityle, sesquicaprylate de xylityle, citrate de zinc, lactate de zinc, néodécanoate de zinc, pyrithione de zinc, ricinoléate de zinc, undécylénate de zinc et

capryloyl/caproyl anhydrométhylglucamide (et) eau.

**13.** Déodorant comprenant un ou les deux composé(s) de formule (I) tels que définis dans la revendication 1.

**14.** Produit à rincer comprenant un ou les deux composé(s) de formule (I) tels que définis dans la revendication 1.

**15.** Produit sans rinçage comprenant un ou les deux composé(s) de formule (I) tels que définis dans la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020160905 A


### Non-patent literature cited in the description


- *Synthesis*, 2003 (15), 2373-2377
- *Journal of the Korean Chemical Society*, 2002, vol. 46 (5)
- *Tetrahedron Letters*, 2015, vol. 56 (15), 1973-1975